# EUROPEAN PATENT APPLICATION

(11) **EP 4 356 731 A1**
(43) Date of publication of application: **24.04.2024**
(21) Application number: 23163832.1
(22) Date of filing: 23.03.2023
(51) Int. Cl.: A01N 1/02

(54) **MEDIUM AND DEVICE FOR COLLECTING, STORING AND TRANSPORTING BIOLOGICAL SAMPLES, AS WELL AS THEIR USE FOR THE STABILIZATION OF EXTRACELLULAR NUCLEIC ACIDS**

(30) Priority: 19.10.2022 RU 2022127083
(71) Applicant: Gra, Olga Alekseevna, Moscow 117418 (RU); Gra, Dmitrii Vadimovich, Moscow 117418 (RU); Seleznev, Viktor Vasilyevich, 171370 Emelyanovo village (RU)
(72) Inventor: Gra, Olga Alekseevna, Moscow 117418 (RU); Gra, Dmitrii Vadimovich, Moscow 117418 (RU); Seleznev, Viktor Vasilyevich, 171370 Emelyanovo village (RU)
(74) Representative: Hoffmann Eitle

(57) **Abstract**

The present invention refers to a medium for collection, storage, and transportation of biological samples, comprising a barrier gel and a stabilizer which in turn comprises at least one cell membrane fixing agent and at least one anticoagulant.

## Description

### FIELD OF THE INVENTION

The present invention refers to the medical field. Particularly, it refers to a medium for collection, storage, and transportation of biological samples, comprising a barrier gel and a stabilizer which in turn comprises at least one cell membrane fixing agent and at least one anticoagulant. Specifically, the present invention pertains to the field of molecular biology, bioorganic chemistry, and genetics, and refers to preanalytical systems for sampling of biological material, containing a medium for stabilization of extracellular nucleic acids for the purpose of storing and transporting biopolymer data for subsequent analysis in clinical diagnostic laboratories and using the results obtained as diagnostic and prognostic markers in various fields of medicine.

### STATE OF THE ART

The analysis of free-circulating extracellular nucleic acids (DNA and RNA) is used for various tasks, including non-invasive prenatal testing - NIPT, for the diagnosis, prognosis and monitoring of the results of cancer treatment, as well as in transplantology, in sports medicine, and even for predicting the course of COVID-19.

In this regard, the following extracellular DNA preservation techniques can be identified:
1. The most common way to prevent genomic DNA release into plasma faction is to collect blood specimen into standard *EDTA or sodium citrate* tubes and store them in a refrigerator, with compulsory centrifugation of the specimen for 6 hours followed by collection of plasma. Once plasma has been collected, it must be frozen prior to DNA extraction. Conversely, if it is necessary to analyze extracellular RNA, a whole blood sample must be centrifuged no later than 1 hour after sampling, and then either RNA extraction should be performed followed by storage at -86°C, or a reverse transcription polymerase chain reaction should be performed to obtain complementary DNA (cDNA), which is more stable, but at the same time, storage and transportation of which must also be carried out in a frozen state. It should be separately noted that even during the indicated time before centrifugation (6 hours in the case of DNA and 1 hour in the case of RNA), significant degradation of extracellular nucleic acids is already observed. The problem with using this method is that most blood drawing points lack necessary equipment and are forced to send samples to centralized laboratories for extraction or analysis of extracellular nucleic acids. At the same time, despite the fact that in most blood drawing points and laboratories it is possible to separate plasma, it must be stored in a frozen state until DNA extraction and subsequent diagnostic manipulations. Conversely, manipulations with extracellular RNA, both RNA extraction and cDNA production, in order to send it frozen to a centralized laboratory, is practically not feasible in medical offices / biomaterial collection points due to the low level of equipment and high requirements for personnel qualification. Therefore, standard EDTA or sodium citrate tubes might not be the perfect or practical solution for the widespread use of extracellular nucleic acids in diagnostic applications, especially for resource-constrained laboratories that lack the necessary equipment for immediate plasma preparation and/or RNA extraction and subsequent storage in the freezer before transporting. Besides, transporting of such specimen requires strict adherence to the cold chain requirements during transportation at negative temperatures, since when defrosting an extracellular DNA/RNA sample can significantly degrade and become unsuitable for further studies. Therefore, these tubes are suitable only for the laboratories which either have no tasks for long-term storage of biomaterial and where the extraction of extracellular DNA/RNA is carried out within one working day, or which have the laboratory equipment enabling to separate plasma / extract RNA and freeze specimen for further transportation.
2. The second method is an improved version of the first method and consists in collecting blood in pink-top tubes similar to the method described in paragraph 1, polyhydric alcohol as a stabilizer and a special (thixotropic) gel that creates a barrier between blood cells and plasma after centrifugation. Since the specific gravity of the thixotropic gel used in tubes is intermediate between the specific gravity of the heavier (blood cells) and lighter (plasma) phases, during centrifugation this gel forms an intermediate layer between the blood cells that remain under the gel and the top plasma layer. The position of the gel after centrifugation is affected by many characteristics of the gel, such as its specific gravity, yield strength, and viscosity. The position of the gel can also be influenced by external conditions such as temperature and centrifugation rate. Additional factors associated with collection of a whole blood sample into a tube may also be involved, such as heparin therapy, low hematocrit, elevated plasma protein, high lipids, and other factors that affect plasma specific gravity. In addition, the type of polymer used to create the gel can also affect its viscosity, density, and other physical properties. This approach to stabilization, storage, and production of extracellular DNA is described in US20200196594 by delta DNA Biosciences Inc. (Canada) and implemented in a HAEM-LOK^{™} blood collection tube. Using a combination of a barrier matrix, a special barrier fluid, and a single centrifugation step, all blood cells (including all "buffy coat" cells) move below the physical barrier to form cell-free plasma. Early removal of all cells prevents the subsequent release of genomic DNA from lysed cells and allows the most efficient extraction of extracellular DNA from the blood plasma fraction that is physically separated from the blood cells. After centrifugation, blood samples in HAEM-LOK^{™} can be stored in the original tube at ambient temperature for 2-3 weeks, simplifying transportation and workflow. Besides, whole blood sample can be stored in this tube for up to 5 days without centrifugation at a temperature ranging from 0°C to +10°C, which allows the sample to be transported to a centralized laboratory from a medical office / biomaterial collection point with a low level of equipment, while transportation requires strict adherence to the cold chain requirements. It should be separately noted, that the shelf life of 5 days and the upper temperature limit of +10 °C are due, particularly, to the absence of cell membrane fixatives or apoptosis inhibitors in the composition of the stabilizing medium of this tube, which does not allow cell stabilization and, as a result, prevents the extraction of genomic DNA from nucleated cells and lysis (hemolysis) of erythrocytes for a longer time and in a wider temperature range.
3. The third method of stabilization followed by separation of plasma from blood cells and extraction of extracellular DNA from the blood plasma fraction is given in patent application WO2017201612. It is based on the differential precipitation method aiming to further concentrate extracellular DNA. It uses the principle originally described by John Lees and Robert Schleif. The authors showed that impact on DNA of a combination of polyethylene glycol and sodium chloride at various concentrations can precipitate DNA of different classes and sizes in different ways. Therefore, high-molecular-weight DNA (genomic DNA) that is extracted from cells at high concentrations of sodium chloride precipitates immediately in the presence of polyethylene glycol. Extracellular DNA, which has a much lower molecular weight, does not precipitate under these conditions, allowing the two classes of DNA to be better separated due to their size. Formulation proposed by the authors of this patent for the stabilization of extracellular DNA does not contain cell membrane fixatives, but only preservatives, anticoagulants and precipitants, which makes it possible to isolate extracellular DNA in a relatively high concentration. This approach to stabilization, storage and isolation of extracellular nucleic acids is implemented in the *cf-DNA & cf RNA Collection & Preservation* blood collection tube from *Norgen Biotek Corp. (Canada),* where the test tube with a biological sample should be stored at a temperature of at least +4°C before analysis to avoid cell hemolysis.
4. The fourth most widely accepted method to prevent the release of genomic DNA into a plasma fraction is to use chemical cell membrane stabilizing agents, such as aldehydes (for instance, formaldehyde or glutaraldehyde), which stabilize nucleated cells by fixing their membrane. This method has a number of disadvantages due to the use of aldehydes as preservatives for the stabilization of extracellular DNA, since they can cause the formation of DNA-protein and DNA-DNA cross-links, which, in turn, can negatively affect subsequent DNA amplification and sequencing. Conversely, in order to neutralize the potentially negative effect of aldehydes on extracellular nucleic acids and the effectiveness of molecular genetic testing, formulations obtained by using this method more often contain additional components that neutralize the effect of aldehydes and their derivatives. A similar approach to preserving extracellular nucleic acids by fixing the mean volume of erythrocytes, preventing leukocyte lysis and, as a result, preventing the release of membrane vesicles into the extracellular space - plasma, is described in international patent application WO2019/079743 A1. The composition developed by its authors is intended to stabilize leukocytes and erythrocytes, preventing the release of nucleic acids and membrane vesicles from cells in a biological specimen. At the same time, due to the presence of an aldehyde "quencher" in the formulation of preservative solution, the composition according to the invention proposed by the authors is suitable for stabilizing biological specimen for the purpose of subsequent analysis by immunoassay, PCR, next generation sequencing (NGS), etc. A similar formulation is used by the authors of patent application US2020/0318161 A1 to stabilize free-circulating extracellular RNA, while the composition of this invention additionally contains nuclease/protease inhibitors and transcription inhibitors that provide RNA stabilization while maintaining the level of RNA expression at the time of blood sampling.

Therefore, the use of test tubes with chemical stabilizers containing precipitants or membrane fixatives, according to the present invention, allows solving the problem of long-term storage and transportation of a whole blood sample in the temperature range from +4 to +37°C, and there is no need for preliminary separation of plasma and freezing, which is suitable for use in the laboratories and medical offices with limited resources.

At the same time, tubes with chemical stabilizers containing precipitants or membrane fixatives, as well as tubes with anticoagulants without and with the addition of a barrier gel, are not intended for storing a sample with extracellular DNA / RNA at temperatures below 0°C, and therefore, to solve logistic tasks during the cold season, regions with a temperate or subpolar climate require either 1) preliminary separation of plasma from blood cells, freezing plasma samples and transporting them frozen with strict adherence to the cold chain requirements, or 2) transporting whole blood samples in test tubes with chemical stabilizers, but also with strict observance of the temperature regime of storage and transportation not lower than +4°C.

Meanwhile, strict adherence to the temperature regime in a narrow temperature range during storage and transportation in combination with the use of thermal recorders (loggers) or thermal indicators results in higher cost of delivering the biomaterial to the centralized laboratory, which can negatively affect the final study cost for a patient, and not always guarantee the safety of the biomaterial. It may lead to an increased risk of repeated blood sampling and a longer waiting time until the result of the study is received, and, therefore, until taking a medical / medical decision, if necessary.

To solve the problem of storing and transporting biological specimen in a wide temperature range, the closest analogue of the technical solution is the medium for storing and transporting blood samples described in the international patent application WO2004032750A1. The medium according to the invention specified in the application includes a barrier gel and a stabilizing solution containing caspase inhibitors as stabilizing agents, proteolytic enzymes belonging to the family of cysteine proteases, and cleaving proteins exclusively after the aspartate (cysteinyl-aspartate-specific proteases). It is also state in the application that the medium contains at least one stabilizing agent in an amount sufficient to prevent or eliminate the occurrence of morphological changes, cell membrane degradation, DNA fragmentation, or loss of cellular function or viability. Caspases are known to regulate many cellular and biochemical changes in dying apoptotic cells. In particular, the DNA fragmentation observed during apoptosis is the result of cleavage by the effector caspase of a complex consisting of the cellular DNAase CAD (caspase-activated DNAase) and its inhibitor ICAD. When the inhibitor is cleaved, active DNAase CAD begins to cleave DNA in the most accessible areas between nucleosomes, which leads to its degradation and the detection of a characteristic "ladder" of DNA fragments of different sizes, observed during apoptosis. In turn, the total inhibition of caspase can prevent the triggering of the caspase-dependent pathway of cell apoptosis and the subsequent release of intracellular nucleic acids and may contribute to less degradation of extracellular DNA due to the lack of release of intracellular DNAases.

Since, in addition to the caspase-dependent pathway of apoptosis, many other mechanisms of cell death are known, in all other cases, including cell apoptosis along the caspase-independent pathway, the stabilizing solution specified by the authors of the international patent application WO2004032750A1, containing one or more caspase inhibitors as stabilizing agents, will not be able to provide preservation and stability of the initial concentration of extracellular nucleic acids, namely DNA and, especially, RNA, in a biological specimen due to the absence of a **universal agent** in the stabilizing solution that can prevent the release of intracellular nucleic acids, as well as the degradation of extracellular nucleic acids due to the lack of release of intracellular nucleoproteases.

Possibly, for this reason, the authors of this international patent application do not position the medium they have developed for stabilizing extracellular nucleic acids, the precise quality of which is necessary for the subsequent research activities that are highly sensitive to the quality of the analyzed specimen, such as NIPT and liquid biopsy, but mainly for the storage of whole blood samples for the conduct of subsequent standard general clinical studies, whilst the temperature range and the period of such storage are not specified in this patent application.

The need to use additional cell stabilizing components, in addition to caspase inhibitors, is pointed out in patent applications US10724074B2 and US10144952B2 of Qiagen GmbH. In particular, authors of these patent applications have shown that caspase inhibitors reduce contamination of the extracellular nucleic acid population with intracellular nucleic acids, in particular, fragmented genomic DNA, which is released from the specimen cells, for instance, from damaged or dying cells. However, only a stabilizing composition including an apoptosis inhibitor and an additional stabilizing agent, such as N,N-dialkylpropanamide, in particular N,N-dimethylpropanamide (DMPA), or butanamide, is effective in maintaining the population of extracellular nucleic acids in the specimen upon taking a biological specimen, and to stabilize the transcriptome of the specimen cells.

The mechanism of action of N,N-dialkylpropanamides, in particular DMPA, and butanamide, has not been fully studied, while there is evidence that some of the butanamide derivatives demonstrate an inhibitory effect on histone deacetylases, being the key enzymes controlling the proliferation or differentiation status of most cells. Conversely, N,N-dialkylpropanamides, and in particular DMPA, are able to stabilize the transcription profile of genes by inhibiting changes in transcript levels, while lymphocyte cells remain intact. Thus, these stabilizing agents used primarily in combination with caspase inhibitors stabilize cells from the inside, preventing their apoptotic death, but do not exert an external stabilizing effect on nucleated cells by fixing their membrane. This explains why the formulations for stabilizing, storing and transporting nucleic acids given in the above-mentioned patent applications US10724074B2 and US10144952B2 provide for a stabilizing effect of up to seven days (mainly 2-3 days) at room temperature without the possibility of refrigeration / freezing or storage for a longer time.

Therefore, despite the progress in the stabilization of extracellular nucleic acids, there is still a need for a formulation and a device for collecting, storing and transporting biological samples, the combination of which would ensure the preservation of extracellular nucleic acids for a long time in a wide range of negative and positive temperatures, and at the same time, it would be suitable for the laboratories with any level of equipment, including medical offices / biomaterial collection points with a minimum instrumentation base.

This invention provide for such formulation (medium) and device, as well as methods of their use for stabilization of extracellular nucleic acids.

### DESCRIPTION OF THE INVENTION

### Brief description of the invention

The present invention to a medium for collection, storage, and transportation of biological samples, comprising a barrier gel and a stabilizer which in turn comprises at least one cell membrane fixing agent and at least one anticoagulant.

Particularly, the present concerns preanalytical systems for sampling of biological material, comprising a stabilizing medium for storing and transporting extracellular nucleic acids for their subsequent analysis and use of the results obtained in clinical practice. The nature of the invention is development of a medium (composition) of a new formulation and a device containing this medium (composition) for collecting, storing and transporting biological samples, as well as new methods for their use, that enable the preservation of extracellular nucleic acids for a long time in a wide temperature range and at the same time are suitable for the laboratories with different equipment level.

Particularly, the first embodiment of the present invention refer to a medium for collection, storage, and transportation of biological samples, comprising a barrier gel and a stabilizer which in turn comprises at least one cell membrane fixing agent and at least one anticoagulant, characterized in that: a) The biological sample comprising the extracellular nucleic acids to be preserved is blood or plasma; and the extracellular nucleic acids are extracellular DNA or RNA; b) the barrier gel comprises thixotropic gel with a density in the range from 1.025 to 1.065 g/cm³, is chemically inert with respect to biological samples, and has sufficient viscosity so that under centrifugal forces in the range of 2000 to 3600 g it will flow and form the desired barrier between plasma and blood cells; c) the cell membrane fixing agent is selected from 2-bromo-2-nitropropane-1,3-diol, dimethylol urea, sodium hydroxymethylglycinate, diazolidinyl urea, dimethyl-dimethylol-hydantoin, imidazolidinyl urea, 1,3,5-tris(hydroxyethyl)-s-triazine, 1,3-bis(hydroxymethyl)-5,5-dimethylimidazolidine-2,4-dione, dimethylol-5,5-dimethylhydantoin, or combinations thereof; releases free aldehyde and has the properties of a preservative; and d) the anticoagulant is selected from ethylene glycol tetraacetic acid (EGTA) and its salts, sodium citrate, ethylenediaminetetraacetic acid (EDTA) and its salts, sodium fluoride or combinations thereof; and acts as an ionic stabilizing and/or chelating agent.

In a preferred embodiment, the fixing agent can mostly be from 0.2% to 63.4% of the total formulation weight.

In a preferred embodiment, the anticoagulant can mostly be from 0.1% to 28.3% of the total formulation mass.

In a preferred embodiment, the stabilizer may not necessarily include a quenching agent in an amount sufficient to react with any free aldehyde that may be present before or after sampling and which is formed from the fixing agent to form a reaction product that will not have a denaturing effect on proteins in biological samples, while the content of the quenching agent can mainly be from 0% to 7.8% by weight of the entire formulation, and wherein: i) a compound including at least one functional group capable of reacting with an electron-deficient functional group of an aldehyde can be used as a quenching agent and ii) ethylenediamine, amino acetic acid, lysine, arginine, adenine, guanine, cytosine, thymine, tryptophan, tyrosine, phenylalanine, ornithine, S-adenosylmethionine, alanine, cysteine, glutamic acid, aspartic acid, histidine, leucine, lysine, proline, serine, threonine, homocysteine, nicotinamide, or combinations thereof can be used as a quenching agent.

In a preferred embodiment, the stabilizer may not necessarily include additional non-ionic stabilizing agents at a concentration of 0% to 18.5% by weight of the entire formulation, which dissolve in an aqueous solution without the formation of ions and can be selected from the group of polyols (polyhydric alcohols), such as sucrose, lactose, trehalose, melibiose, mannitol, or combinations thereof.

In a preferred embodiment, the stabilizer may not necessarily include enzyme inhibitors - nucleases and / or proteases, including inhibitors of metabolic enzymes and inhibitors of phosphatase, which prevent the degradation of nucleic acids and ensure their stabilization, as well as inhibitors of apoptosis, including caspase inhibitors, including in combination with N,N-dialkylpropanamide, for example, with N,N-dimethylpropanamide (DMPA), or butanamide, which prevent cell apoptosis through a caspase-dependent pathway, and transcription inhibitors, which are designed to maintain the basic level of RNA expression at the time of blood sampling by inhibiting the synthesis of new RNA transcripts, including mRNA, microRNA, IncRNA, piRNA, YRNA, circRNA and other ncRNA: i) enzyme inhibitors can be selected from the groups of compounds, including but not limited to diethyl pyrocarbonate, ethanol, auryl tricarboxylic acid (ATA), glyceraldehydes, formamide, bentonite, ammonium sulfate, dithiothreitol (DTT), beta-mercaptoethanol, cysteine, dithioerythritol, tris(2-carboxyethyl)phosphine hydrochloride or combinations thereof; ii) inhibitors of metabolic enzymes can be selected from the groups of compounds, including but not limited to glyceraldehyde, dihydroxyacetone phosphate, glyceraldehyde-3-phosphate, 1,3-bisphosphoglycerate, 3-phosphoglycerate, 2 phosphoglycerate, phosphoenolpyruvate, pyruvate and glycerate dihydroxyacetate, potassium oxalate or combinations thereof; iii) protease inhibitors can be selected from the groups of compounds, including but not limited to antipain, chymostatin, elastatinal, phenylmethylsulfonyl fluoride (PMSF), APMSF, TLCK, TPCK, leupeptin, soybean trypsin inhibitor, indoleacetic acid (IAA), E64, pepstatin, 1,10-phenanthroline, phosphoramodone, amastatin, bestatin, diprotin A, diprotin B, alpha-2-macroglobulin, pancreatic protease inhibitor, ovostatin, cystatin, protease inhibitor, or combinations thereof; iv) phosphatase inhibitors can be selected from the groups of compounds, including but not limited to caliculin A, nodularin, NIPP-1, microcystin LR, tautomycin, okadaic (okadaic) acid, cantharidin, fostriyetsin, endothall, cyclosporin A, FK 506/immunophilin complexes, cypermethrin, deltamethrin, fenvalerate, defostatin, mpV (pic) DMHV, sodium orthovanadate, or combinations thereof; v) the content of enzyme inhibitors - nucleases and / or proteases, including inhibitors of metabolic enzymes and phosphatase inhibitors, can mainly range from 0% to 25.8% by weight of the entire formulation.

In a preferred embodiment, the stabilizer may optionally contain, at a concentration of 0% to 5.4%, one or more cell permeability enhancing agents, which may be selected from the following compounds or combinations thereof: DMSO (dimethyl sulfoxide), ethylene glycol, glycerin, cellosolves, ethylene glycol dimethyl ether, phenoxyethanol, Triton X 100, Triton X 705 (non-ionic detergent), 1-methyl-2-pyrrolidinone, Tween 20, Tween 40 (non-ionic detergent), Brij 35 (non-ionic detergent), polyoxyethylene ester (Polyox), monensin, monactin, pentachlorophenol, 2,4-dinitrophenol, saponin, SDS (sodium dodecyl sulfate).

In a preferred embodiment, the stabilizer may optionally contain the following compounds or combinations thereof at a concentration of 0% to 6.2%: i) proteins such as: biotin, albumins: egg, bovine, including bovine serum albumin - BSA, gelatin and similar compounds; ii) additional nucleic acid stabilizers such as: guanidine hydrochloride, polycations such as polyethyleneimine and similar compounds; iii) antioxidants/reductants such as Trolox, α-tocopherol, nicotinamide and similar compounds; iv) nucleic acid dyes such as: DAPI (diamidino-2-phenylindole), propidium iodide, fluorescein diacetate and similar compounds; v) antibiotics and other additional components, including but not limited to doxycycline, sulfasalazine, curcumin, 6-aminocaproic acid, minocycline, chitosan, phytic acid, β-sitosterol, c-AMP, polylysine, biochanin A, demeclocycline, chlortetracycline, oxytetracycline, cyclohexamide, rifampicin, soy milk, suramin, N-butyric acid, penicillamine, N-acetylcysteine, benzamidine, 2 aminoethylbenzylsulfonyl fluoride (AEBSF).

In a preferred embodiment, the pH of the stabilizer can be, depending on the combination of its constituent agents and additional components, from 4 to 10, mostly from 6 to 8.

The second embodiment of the present invention refers to a device for collecting, storing and transporting biological specimen containing the medium of the present invention, which comprises a test tube with the medium of the present invention, provided that the stabilizer containing the cell membrane fixing agent, which is part of the medium of the present invention, is located above the thixotropic gel and / or the specified thixotropic gel is located under the specified stabilizer.

In a preferred embodiment, the device comprises vacuum tubes, including sterile ones, which may contain a vacuum intended for aspiration of a biological sample, while vacuum tubes may mostly have, without the limitation, a nominal capacity of 9.0 ml, 8.5 ml, 8.0 ml, 7.5 ml, 7.0 ml (size 16×100 mm), 6.0 ml, 5.5 ml, 5.0 ml (size 13×100 mm), 2.0 ml, 2.5 ml, 3.0 ml, 3.5 ml, 4.0 ml, 4.5 ml (13×75 mm).

In a preferred embodiment, the content of the separation gel is mostly from 0.3 g to 2.5 g per tube, without the limitation.

The third embodiment of the present invention refers to the use of the device of the invention, which comprises the medium of the invention, for stabilizing extracellular nucleic acids for up to 15 days at temperatures ranging from -20°C to +37°C and up to 12 months at temperatures ranging from -86°C to -20°C.

In a preferred embodiment, the present invention refers to the use of the device of the invention characterized in that it is carried out through the interaction of a biological sample located in the device of the invention, with a cell membrane fixing agent that is part of the stabilizing medium solution of the invention, and a separating gel, which after centrifugation for 10-30 minutes at 2000-3600g creates a separating barrier between the supernatant and cells, mainly plasma and blood cells, making the nucleic acids in the plasma sample suitable for extraction and subsequent analysis, including but not limited to PCR and sequencing.

In a preferred embodiment, the present invention refers to the use of the device of the invention characterized by taking a biological sample with the possibility of storage and transportation without centrifugation up to 24 hours after taking at a temperature ranging from +4°C to +37°C, followed by centrifugation and further storage and transportation from 5 to 15 days at temperatures from -20°C to +37°C with the possibility of repeated freezing / thawing of the sample after centrifugation with storage at temperatures from -86°C to -20°C for up to 12 months.

In a preferred embodiment, the present invention refers to the use of the device of the invention characterized by the possibility of storing and transporting a biological sample without centrifugation from 5 to 15 days after taking at a temperature ranging from +4°C to +37°C and centrifugation immediately before the collection of the supernatant, mainly plasma, to remove cells, extracellular structures and cellular debris to obtain cell-free plasma.

In a preferred embodiment, the present invention refers to the use of the device of the invention characterized by the possibility of storing and transporting a biological sample without centrifugation from 5 to 15 days after taking at a temperature ranging from +4°C to +37°C while preserving extracellular nucleic acids, as well as circulating cells and / or extracellular structures, if necessary.

The essence of this invention lies in the development of new medium composition and a device containing this medium for collection and subsequent storage and for transportation of biological samples for a long time in a wide temperature range, as well as development of new ways of using them to stabilize nucleic acids in laboratories with different levels of equipment.

Technical result of application of this invention is the following:
- Stabilization of a cell membrane due to cross-links and, as a result, fixation of the cell membrane preventing its hemolysis, regardless of the nature of intracellular apoptotic processes and cell maturity. As a result, fixed cells do not release intracellular DNA or RNA not only during storage before centrifugation, ensuring the preservation of the concentration of nucleic acids at the time of blood sampling, but also during centrifugation due to the fixed membrane, since they become more resistant to mechanical stress, which allows not only to move all blood cells intact under the gel barrier, obtaining cell-free plasma, but also to avoid release into plasma of intracellular nucleic acids during centrifugation.
- Increased stabilization time for extracellular nucleic acids in a wide temperature range: up to 15 days at temperatures ranging from -20°C to +37°C and up to 12 months at temperatures ranging from -86°C to -20°C, which enables to store and transport a specimen, which is predominantly a blood sample with extracellular nucleic acids that need to be preserved, under almost any conditions and resources available to the laboratory.
- The invention allows centrifugation with centrifugal acceleration already from 2000 to 3600g in wider time ranges from 10 to 30 minutes without mechanical injury to the cell membrane, while in the prior art it is recommended to use a fundamentally gentler centrifugation mode: 10 minutes at 2000g in order to avoid additional release of nucleic acids from cells due to their traumatization. Conversely, a longer centrifugation with a higher acceleration makes it possible to isolate residual blood cells, extracellular structures and cellular debris, which, in turn, makes it possible, after plasma collection, not to carry out a second round of centrifugation at 13000-16000g for 10-20 minutes immediately prior to the nucleic acid extraction phase. This technical result is relevant for the laboratories with an extensive instrument base, a large flow of research and the need to reduce the average time for sample preparation and nucleic acid extraction.
- The invention allows efficient centrifugation of platelets without degranulation with simultaneous adsorption of platelets by the gel, which completely excludes the release of mitochondrial DNA into the plasma planned for further analysis. This allows, even with the minimum recommended centrifugation time of 10 minutes and an acceleration of 2000g, to isolate all cells from the plasma, including platelets and "buffy coat" cells, and to obtain cell-free plasma without impurities of mitochondrial and apoptotic DNA, introduced additionally from cells after blood sampling. This technical result enables to reduce equipment requirements to a minimum and to carry out centrifugation in medical offices / biomaterial collection points with limited resources by using compact laboratory centrifuges.
- Higher concentration of extracellular DNA and RNA, which is detected when extracting DNA / RNA after centrifugation from blood samples in tubes according to the invention with an anticoagulant (for example, EDTA tubes), cell membrane fixative and gel compared to ***EDTA*** tubes with without / with barrier gel and specialized tubes for stabilization of extracellular nucleic acids, in particular, with ***Cell-Free DNA BCT^{®}*** tubes manufactured by Streck (USA) with anticoagulant and cell membrane fixative without gel. Studies have shown that
   ✔ in case of DNA - this is an increase of up to 2.4 / 2.4 and 1.7 times, respectively;
   ✔ in case of RNA - this is an increase on average up to 464 / 567 and 36 times, respectively.
- Higher concentration of extracellular DNA and RNA, which is detected at extraction of DNA / RNA after centrifugation and 1 freeze / thaw cycle of a blood sample in tubes according to the invention with an anticoagulant (for example, EDTA tubes), cell membrane fixative and gel compared to ***EDTA*** tubes without / with barrier gel and specialized tubes for stabilization of extracellular nucleic acids, in particular, with ***RNA Complete BCT^{®}*** tubes manufactured by Streck (USA) with anticoagulant and cell membrane fixative without gel without freezing cycles.
- Studies have shown that
   ✔ in case of DNA - this is an increase of up to 2.9 / 3.0 and 2.0 times, respectively;
   ✔ in case of RNA - this is an increase on average up to 1013 / 1317 and 76 times, respectively.

So, in a preferred embodiment, the present invention ensuring the above-mentioned technical result covers the following:
A medium for collection, storage, and transportation of biological samples,
- where the biological sample containing the extracellular nucleic acids to be preserved is blood or plasma;
- where extracellular nucleic acids are extracellular DNA or RNA;
   containing barrier gel
- where a thixotropic gel is used as a barrier gel;
- where thixotropic gel has a density in the range from 1.025 to 1.065 g/cm³ and is chemically inert with respect to biological samples;
- where the thixotropic gel has sufficient viscosity so that under centrifugal forces in the range of 2000 to 3600 g it will flow and form the desired barrier between plasma and blood cells;
   and stabilizer containing at least 1 cell membrane fixing agent
- where 2-bromo-2-nitropropane-1,3-diol, dimethylol urea, sodium hydroxymethylglycinate, diazolidinyl urea, dimethyl-dimethylol-hydantoin, imidazolidinyl urea, 1,3,5-tris(hydroxyethyl)-s-triazine, 1,3-bis(hydroxymethyl)-5,5-dimethylimidazolidine-2,4-dione, dimethylol-5,5-dimethylhydantoin, or combinations thereof are used as a cell membrane fixing agent that is a part of the stabilizer;
- where the fixing agent releases free aldehyde and has the properties of a preservative,
   and at least 1 anticoagulant
- where ethylene glycol tetraacetic acid (EGTA) and its salts, sodium citrate, ethylenediaminetetraacetic acid (EDTA) and its salts, sodium fluoride or combinations of them are used as an anticoagulant, which is part of the stabilizer;
- where the anticoagulant can act as an ionic stabilizing and/or chelating agent.

In a preferred embodiment, the fixing agent can mostly be from 0.2% to 63.4% of the total formulation weight.

In a preferred embodiment, the anticoagulant can mostly be from 0.1% to 28.3% of the total formulation mass.

In a preferred embodiment, the stabilizer may not necessarily include a quenching agent in an amount sufficient to react with any free aldehyde that may be present before or after sampling and which is formed from the fixing agent to form a reaction product that will not have a denaturing effect on proteins in biological samples, while the content of the quenching agent can mainly be from 0% to 7.8% by weight of the entire formulation,
- where a compound including at least one functional group capable of reacting with an electron-deficient functional group of an aldehyde can be used as a quenching agent;
- where ethylenediamine, aminoacetic acid, lysine, arginine, adenine, guanine, cytosine, thymine, tryptophan, tyrosine, phenylalanine, ornithine, S-adenosylmethionine, alanine, cysteine, glutamic acid, aspartic acid, histidine, leucine, lysine, proline, serine, threonine, homocysteine, nicotinamide, or combinations thereof can be used as a quenching agent.

In a preferred embodiment, the stabilizer may not necessarily include additional non-ionic stabilizing agents at a concentration of 0% to 18.5% by weight of the entire formulation, which dissolve in an aqueous solution without the formation of ions and can be selected from the group of polyols (polyhydric alcohols), such as sucrose, lactose, trehalose, melibiose, mannitol, or combinations thereof.

In a preferred embodiment, the stabilizer may not necessarily include enzyme inhibitors - nucleases and / or proteases, including inhibitors of metabolic enzymes and inhibitors of phosphatase, which prevent the degradation of nucleic acids and ensure their stabilization, as well as inhibitors of apoptosis, including caspase inhibitors, including in combination with N,N-dialkylpropanamide, for example, with N,N-dimethylpropanamide (DMPA), or butanamide, which prevent cell apoptosis through a caspase-dependent pathway, and transcription inhibitors, which are designed to maintain the basic level of RNA expression at the time of blood sampling by inhibiting the synthesis of new RNA transcripts, including mRNA, microRNA, IncRNA, piRNA, YRNA, circRNA and other ncRNA:
- enzyme inhibitors can be selected from the groups of compounds, including but not limited to diethyl pyrocarbonate, ethanol, auryl tricarboxylic acid (ATA), glyceraldehydes, formamide, bentonite, ammonium sulfate, dithiothreitol (DTT), beta-mercaptoethanol, cysteine, dithioerythritol, tris(2-carboxyethyl)phosphine hydrochloride or combinations thereof;
- inhibitors of metabolic enzymes can be selected from the groups of compounds, including but not limited to glyceraldehyde, dihydroxyacetone phosphate, glyceraldehyde-3-phosphate, 1,3-bisphosphoglycerate, 3-phosphoglycerate, 2-phosphoglycerate, phosphoenolpyruvate, pyruvate and glycerate dihydroxyacetate, potassium oxalate or combinations thereof;
- protease inhibitors can be selected from the groups of compounds, including but not limited to antipain, chymostatin, elastatinal, phenylmethylsulfonyl fluoride (PMSF), APMSF, TLCK, TPCK, leupeptin, soybean trypsin inhibitor, indoleacetic acid (IAA), E64, pepstatin, 1,10-phenanthroline, phosphoramodone, amastatin, bestatin, diprotin A, diprotin B, alpha-2-macroglobulin, pancreatic protease inhibitor, ovostatin, cystatin, protease inhibitor, or combinations thereof;
- phosphatase inhibitors can be selected from the groups of compounds, including but not limited to caliculin A, nodularin, NIPP-1, microcystin LR, tautomycin, okadaic (okadaic) acid, cantharidin, fostriyetsin, endothall, cyclosporin A, FK 506/immunophilin complexes, cypermethrin, deltamethrin, fenvalerate, defostatin, mpV (pic) DMHV, sodium orthovanadate, or combinations thereof;
- the content of enzyme inhibitors - nucleases and / or proteases, including inhibitors of metabolic enzymes and phosphatase inhibitors, can mainly range from 0% to 25.8% by weight of the entire formulation.

In a preferred embodiment, the stabilizer may optionally contain, at a concentration of 0% to 5.4%, one or more cell permeability enhancing agents, which may be selected from the following compounds or combinations thereof: DMSO (dimethyl sulfoxide), ethylene glycol, glycerin, cellosolves, ethylene glycol dimethyl ether, phenoxyethanol, Triton X 100, Triton X 705 (non-ionic detergent), 1-methyl-2-pyrrolidinone, Tween 20, Tween 40 (non-ionic detergent), Brij 35 (non-ionic detergent), polyoxyethylene ester (Polyox), monensin, monactin, pentachlorophenol, 2,4-dinitrophenol, saponin, SDS (sodium dodecyl sulfate).

In a preferred embodiment, the stabilizer may optionally contain the following compounds or combinations thereof at a concentration of 0% to 6.2%:
- proteins such as: biotin, albumins: egg, bovine, including bovine serum albumin - BSA, gelatin and similar compounds;
- additional nucleic acid stabilizers such as: guanidine hydrochloride, polycations such as polyethyleneimine and similar compounds;
- antioxidants/reductants such as Trolox, α-tocopherol, nicotinamide and similar compounds;
- nucleic acid dyes such as: DAPI (diamidino-2-phenylindole), propidium iodide, fluorescein diacetate and similar compounds;
- antibiotics and other additional components, including but not limited to doxycycline, sulfasalazine, curcumin, 6-aminocaproic acid, minocycline, chitosan, phytic acid, β-sitosterol, c-AMP, polylysine, biochanin A, demeclocycline, chlortetracycline, oxytetracycline, cyclohexamide, rifampicin, soy milk, suramin, N-butyric acid, penicillamine, N-acetylcysteine, benzamidine, 2-aminoethylbenzylsulfonyl fluoride (AEBSF).

In a preferred embodiment, the pH of the stabilizer can be, depending on the combination of its constituent agents and additional components, from 4 to 10, mostly from 6 to 8.

Device for collecting, storing and transporting biological specimen containing the medium according to the present invention, represents a test tube with the medium according to the present invention, provided that the stabilizer containing the cell membrane fixing agent, which is part of the medium according to the present invention, is located above the thixotropic gel and / or the specified thixotropic gel is located under the specified stabilizer.

In a preferred embodiment, the device according to In a preferred embodiment, represents vacuum tubes, including sterile ones, which may contain a vacuum intended for aspiration of a biological sample, while vacuum tubes may mostly have, without the limitation, a nominal capacity of 9.0 ml, 8.5 ml, 8.0 ml, 7.5 ml, 7.0 ml (size 16×100 mm), 6.0 ml, 5.5 ml, 5.0 ml (size 13×100 mm), 2.0 ml, 2.5 ml, 3.0 ml, 3.5 ml, 4.0 ml, 4.5 ml (13×75 mm).

In a preferred embodiment, the device according to the present invention, is characterize din that the content of the separation gel is mostly from 0.3 g to 2.5 g per tube, without the limitation. Use of device of the present invention, containing the medium of the present invention, for stabilizing extracellular nucleic acids for up to 15 days at temperatures ranging from -20°C to +37°C and up to 12 months at temperatures ranging from -86°C to -20°C.

A possible application of the device of the invention is where the device is used through the interaction of a biological sample located in the device according to the present invention, with a cell membrane fixing agent that is part of the stabilizing medium solution according to the present invention, and a separating gel, which after centrifugation for 10-30 minutes at 2000-3600g creates a separating barrier between the supernatant and cells, mainly plasma and blood cells, making the nucleic acids in the plasma sample suitable for extraction and subsequent analysis, including but not limited to PCR and sequencing;

A possible application of the device of the invention is where the use includes taking a biological sample with the possibility of storage and transportation without centrifugation up to 24 hours after taking at a temperature ranging from +4°C to +37°C, followed by centrifugation and further storage and transportation from 5 to 15 days at temperatures from -20°C to +37°C with the possibility of repeated freezing / thawing of the sample after centrifugation with storage at temperatures from -86°C to -20°C for up to 12 months.

A possible application of the device of the invention is where its use provides for the possibility of storing and transporting a biological sample without centrifugation from 5 to 15 days after taking at a temperature ranging from +4°C to +37°C and centrifugation immediately before the collection of the supernatant, mainly plasma, to remove cells, extracellular structures and cellular debris to obtain cell-free plasma.

A possible application of the device of the invention is where its use provides for the possibility of storing and transporting a biological sample without centrifugation from 5 to 15 days after taking at a temperature ranging from +4°C to +37°C while preserving extracellular nucleic acids, as well as circulating cells and / or extracellular structures, if necessary.

The main aspect of the invention is a medium for collecting, storing and transporting biological samples, containing a thixotropic barrier gel, a stabilizer comprising at least one cell membrane fixing agent, and an anticoagulant. The cell membrane fixing agent at a preferred concentration of 0.2% to 63.4% may be selected from the following groups of compounds or a combination thereof: *2-bromo-2-nitropropane-1,3-diol, dimethylol urea, sodium hydroxymethylglycinate, diazolidinyl urea, dimethyl-dimethylol-hydantoin, imidazolidinyl urea, 1,3,5-tris(hydroxyethyl)-s-triazine, 1,3-bis(hydroxymethyl)-5,5-dimethylimidazolidine-2,4-dione, dimethylol-5,5-dimethylhydantoin,* while the fixing effect of the selected compounds is mainly associated with their reaction with membrane proteins, namely with the formation of covalent bonds both within proteins and between them. It results to rigidness of the cell membrane and, therefore, resists degradation and mechanical stress much better, in contrast to the action of caspase inhibitors indicated in the international patent application WO2004032750A1*,* as well as in patents for inventions US10724074B2 and US10144952B2***,*** which are not chemically "fix" the cell membrane, making it more rigid by crosslinking membrane proteins, and indirectly, by inhibiting caspase, they prevent cell apoptosis along the caspase-dependent pathway and the release of intracellular DNA, as well as the degradation of extracellular DNA.

According to the main aspect of the invention, the stabilizer of the Medium includes at least 1 anticoagulant,
- where ethylene glycol tetra acetic acid (EGTA) and its salts, sodium citrate, ethylenediaminetetraacetic acid (EDTA) and its salts, sodium fluoride or combinations thereof are used as an anticoagulant, being part of the stabilizer;
- where the anticoagulant can act as an ionic stabilizing and/or chelating agent.

According to the main aspect of the invention, medium for collecting, storing and transporting biological samples, in addition to a stabilizer, comprising at least one cell membrane fixing agent, which is able to effectively prevent the release of genomic DNA from nucleated cells (in furtherance distinguishing this Medium from a thixotropic gel based composition according to patent application US20200196594**),** also contains a thixotropic barrier gel, which distinguishes this Medium from compositions based on a cell membrane fixing agent presented in patent applications EP1413874A1 and WO2019/079743A1***.*** The use of a thixotropic gel barrier as a barrier matrix makes it possible to separate cells from plasma and thereby prevent the subsequent release of genomic DNA from lysed cells.

It should be separately noted that according to the main aspect of this invention a thixotropic gel as a barrier gel in the formulation of the Medium for collecting, storing and transporting biological samples is used mainly in an amount of 0.3 g to 2.5 g, including but not limited to olefin, polyester, polyamide gel. In addition, polyesters, denatured collagens, polypropylenes, polysiloxanes such as dimethylpolysiloxane and ethyltriethoxysilane, hydrocarbon gel materials such as polybutene, and combinations thereof can be used as components of the thixotropic gel. At the same time, the thixotropic gel, also referred herein to as a barrier gel, has a density in the approximate range from 1.025 to 1.065 g/cm³ and is chemically inert with respect to biological specimen, in particular, to blood components, without the limitation.

Therefore, the proposed formulation of the Medium for collecting, storing and transporting biological samples according to the main aspect of this invention provides a technical result, namely, stabilization due to cross-links and, as a result, fixation of the cell membrane, which prevents the hemolysis (of which during apoptosis). Conversely, during centrifugation, cells with a fixed membrane become more resistant to mechanical stress, which allows not only to move all blood cells intact under the gel barrier, obtaining cell-free plasma, but also to avoid release into plasma during centrifugation of intracellular nucleic acids, including genomic DNA, which can significantly contribute to the total amount of extracellular nucleic acids in the analyzed specimen (increase the amount of extracellular nucleic acids relative to the amount that was present in the specimen at the time of blood sampling) and, as a result, significantly reduce the analysis sensitivity, for example, violating the ratio fetal/maternal or genomic/tumor DNA. In particular, this also applies to the release of mitochondrial DNA from platelets, which occurs after the activation and subsequent degranulation of these cells. In this case, the platelet activator can be a mechanical effect, including centrifugation, in which excessive acceleration increases the percentage of activated platelets, which is accompanied by their premature degranulation. The presence of a membrane-fixing agent makes the platelet membrane more resistant to mechanical stress, preventing their premature activation and release of extracellular mitochondrial DNA. According to the main aspect of this invention, medium for collecting, storing and transporting biological samples may also contain additional components that enhance the stabilizing effect. Thus, for example, according to the main aspect of this invention the composition of the stabilizer in the Medium may optionally include a quenching agent in an amount sufficient to react with any free formaldehyde that can be formed from the cell membrane fixing agent with the formation of a reaction product, which will not have a denaturing effect on proteins in biological samples. In turn, as a quenching agent, a compound including at least one functional group capable of reacting with an electron-deficient functional group of formaldehyde can be used. It could be selected without the limitation from the group consisting of an amino compound that reacts with formaldehyde to form methylol, and/ or an imine Schiff base and/or a cis-diol compound that reacts with formaldehyde to form a cyclic acetal or a combination thereof.

The quenching agent at a preferred concentration of 0% to 7.8% may be selected from the following, without the limitation, compounds/groups of compounds or combinations thereof:
- alkylamines, polyamines, amino acids, primary amines, secondary amines, ammonium salts;
- ethylenediamine, urea, glycine, lysine, arginine, adenine, guanine, cytosine, thymine, spermidine, tryptophan, tyrosine, phenylalanine, ornithine, S-adenosylmethionine, aspartate, glutamine, alanine, cysteine, glutamic acid, aspartic acid, histidine, leucine, lysine, proline, serine, threonine, homocysteine, nicotinamide.

Moreover, according to the main aspect of this invention the stabilizer of the Medium may optionally include additional non-ionic stabilizing agents that dissolve in an aqueous solution without the formation of ions. Nonionic stabilizing agents at a preferred concentration of 0% to 18.5% may be selected from the group of polyols (polyhydric alcohols) such as, but not limited to, sucrose, lactose, trehalose, melibiose, mannitol, inositol, or combinations thereof.

Moreover, according to the main aspect of this invention the stabilizer of the Medium may optionally include diluting agents at a preferred concentration of 0% to 15%, including iso- or hypertonic solutions of metrizamide, an iodinated organic compound having a lipophilic substituent, or mammalian cell culture media, which prevent or reverse swelling of nucleated cells, which in turn, increases their buoyant density, thereby increasing the purity of the plasma. Additionally, according to the main aspect of this invention the stabilizer of the Medium may optionally include genomic DNA precipitating agents in a preferred concentration from 0% to 34.5%, such as polyethylene glycol (PEG with various molecular weights, which are known in this art) or other polymers, including cyclodextrins, including but not limited to: *polyvinylpyrrolidone (PVP with various molecular weights that are known in the art), magnesium gluconate, methylcellulose (MC), ethylcellulose (EC), hydroxyethylcellulose (HEC )*, *hydroxypropyl cellulose (HPC), dextrins (with various molecular weights as known in this art), dextrans (with various molecular weights), polyethylene oxide, polyethyloxazoline, ficolls (with various molecular weights), α-cyclodextrin, fl-cyclodextrin, Y -cyclodextrin, gelatins, sugars (not limited), hydroxypropyl methylcellulose, hydroxyethyl methylcellulose.*

Also, according to the main aspect of this invention the stabilizer of the Medium may optionally include inhibitors of enzymes - nucleases and / or proteases in concentrations from 0% to 25.8%, including inhibitors of metabolic enzymes and phosphatase inhibitors, which prevent the degradation of nucleic acids and ensure their stabilization, as well as inhibitors of apoptosis, including caspase inhibitors, including in combination with N,N-dialkylpropanamide, for example, with N,N-dimethylpropanamide (DMPA), or butanamide, which prevent cell apoptosis in a caspase-dependent manner, and transcription inhibitors, which are designed to maintain a baseline level of RNA expression at the time of blood sampling by inhibiting the synthesis of new RNA transcripts (including mRNA, miRNA, IncRNA, piRNA, YRNA, circRNA and other ncRNA).
- Enzyme inhibitors can be selected from the following groups of compounds, including without the limitation diethyl pyrocarbonate, ethanol, glyceraldehydes, sodium fluoride, ethylenediaminetetraacetic acid (EDTA), formamide, aurine tricarboxylic acid (ATA), vanadyl-ribonucleoside complexes, heparin, bentonite, ammonium sulfate, dithiothreitol (DTT), beta-mercaptoethanol, cysteine, dithioerythritol, but not limited to tris(2-carboxyethyl)phosphene hydrochloride or combinations thereof.
- Metabolic enzyme inhibitors may be selected from the following groups of compounds, including but not limited to glyceraldehyde, dihydroxyacetone phosphate, glyceraldehyde-3-phosphate, 1,3-bisphosphoglycerate, 3-phosphoglycerate, 2-phosphoglycerate, phosphoenolpyruvate, pyruvate and glycerate dihydroxyacetate, sodium fluoride, potassium oxalate or combinations thereof.
- Protease inhibitors can be selected from the following groups of compounds, including without the limitation antipain, aprotinin, chymostatin, elastatinal, phenylmethylsulfonyl fluoride (PMSF), APMSF, TLCK, TPCK, leupeptin, soybean trypsin inhibitor, indoleacetic acid (IAA), E64, pepstatin, VdLPFFVdL, EDTA, 1,10-phenanthroline, phosphoramodone, amastatin, bestatin, diprotin A, diprotin B, alpha-2-macroglobulin, lima bean trypsin inhibitor, pancreatic protease inhibitor, egg white ovostatin, egg white cystatin, soy protease inhibitor or a combinations thereof.
- Phosphatase inhibitors can be selected from the following groups of compounds, including without the limitation caliculin A, nodularin, NIPP-1, microcystin LR, tautomycin, okadaic acid, cantharidin, fostriycin, endothallium, cyclosporin A, FK 506/immunophilin complexes, cypermethrin, deltamethrin, fenvalerate, defostatin, mpV (pic) DMHV, sodium orthovanadate, or combinations thereof.

Additionally, according to the main aspect of this invention the formulation of the stabilizer included in the Medium is optional, but may include one or more agents that increase cell permeability in concentrations from 0% to 5.4%, which can be selected from the following compounds or combinations thereof: DMSO (dimethyl sulfoxide), ethylene glycol, glycerin, cellosolve, ethylene glycol dimethyl ether, phenoxyethanol, Triton X 100, Triton X 705 (non-ionic detergent), 1-methyl-2-pyrrolidinone, Tween 20, Tween 40 (non-ionic detergent), Brij 35 (non-ionic detergent), polyoxyethylene ether (Polyox), monensin, monactin, pentachlorophenol, 2,4-dinitrophenol, saponin, SDS (sodium dodecyl sulfate).

Also, the stabilizer, which is part of the Medium according to the main aspect of this invention, is optional, but may contain the following components in a concentration of from 0% to 6.2%, including but not limited to:
- proteins such as: biotin, albumins (egg, bovine, including bovine serum albumin - BSA), gelatin and similar compounds;
- additional nucleic acid stabilizers such as: guanidine hydrochloride, polycations such as polyethyleneimine and similar compounds;
- antioxidants/reducing agents such as Trolox, α-tocopherol, nicotinamide and similar compounds;
- nucleic acid stains such as: DAPI (diamidino-2-phenylindole), propidium iodide, fluorescein diacetate and similar compounds;
- antibiotics and other additional components, such as: doxycycline, sulfasalazine, curcumin, 6-aminocaproic acid, minocycline, chitosan, phytic acid, β-sitosterol, c-AMP, polylysine, biochanin A, demeclocycline, chlortetracycline, oxytetracycline, cyclohexamide, rifampicin, soy milk, suramin, N-butyric acid, penicillamine, N-acetylcysteine, benzamidine, 2-aminoethylbenzylsulfonyl fluoride (AEBSF).

According to the main aspect of this invention, pH of the stabilizer, which is part of the Medium, can be, depending on the combination of its constituent agents and additional components, from 4 to 10, mostly from 6 to 8.

The second aspect of this invention is a device for collecting, storing and transporting biological samples, containing the Medium according to the main aspect of the invention. The device mainly consists of vacuum tubes with a thixotropic barrier gel and a stabilizer containing at least one cell membrane fixing agent and located above the barrier gel. The tubes can be of any nominal capacity and can have any shell form that allows centrifugation and/or sedimentation of blood cells, of which in combination with genomic DNA, or use other concentration methods that ensure extraction of extracellular nucleic acids, of which from circulating cells and /or extracellular structures.

It should be separately noted that biological specimen containing the nucleic acids to be preserved is a biological fluid. In turn, the biological fluid is predominantly, without the limitation, blood, plasma, serum, urine, saliva, stool, breast milk, tears, sweat, cerebrospinal fluid, synovial fluid, semen, vaginal fluid, ascitic fluid, amniotic fluid or cell culture media. Collection of a biological fluid in the Device for collecting, storing and transporting biological samples according to the second aspect of this invention can be carried out both by venipuncture using standard phlebotomy equipment for taking whole blood from a vein, and by introducing (adding) an appropriate volume of biological fluid into the Device with Medium according to the main aspect of this invention. At the same time, the nucleic acids that need to be preserved are mainly located, but not limited to, in blood plasma.

In turn, the presence in the proposed Device for collecting, storing and transporting biological samples according to the second aspect of this invention, containing the Medium according to the main aspect of this invention, and a thixotropic barrier gel in combination with a cell membrane fixing agent ensures not only the effective centrifugation of platelets without degranulation, but also the adsorption of platelets by gel, which completely excludes the release of mitochondrial DNA into the plasma planned for further analysis. This allows, even with the minimum recommended centrifugation time of 10 minutes and an acceleration of 2000 g, to isolate all cells from plasma, including platelets and cells of the "buffy coat", and to obtain cell-free plasma without impurities of mitochondrial and apoptotic DNA, introduced additionally from cells after blood sampling. This technical result allows reducing the equipment requirements to a minimum and carrying out centrifugation in medical offices / biomaterial collection points with limited resources using compact laboratory centrifuges.

At the same time, presence of the cell membrane fixative in the stabilizer contained in the proposed Device for collecting, storing and transporting biological samples according to the second aspect of this invention, with the Medium according to the main aspect of this invention, allows centrifugation with centrifugal acceleration already from 2000 to 3600 g in wider time ranges from 10 up to 30 minutes without any mechanical injury to the cell membrane, while in the case of taking whole blood into a stabilizer without a cell membrane fixing agent, the authors of patent US20200196594 recommend using a fundamentally more gentle centrifugation mode: 10 minutes at 2000g to avoid additional release of nucleic acids from cells due to their possible traumatization. In turn, longer centrifugation with higher acceleration makes it possible to isolate residual blood cells, extracellular structures and cellular debris, which, in turn, makes it possible, after plasma collection, not to carry out a second round of centrifugation at 13000-16000g for 10-20 minutes immediately prior to the nucleic acid extraction step. This technical result is relevant for laboratories with an extensive instrumentation base, a large flow of research and the need to reduce the average time for sample preparation and nucleic acid extraction.

The third aspect of this invention is the use of the device according to the invention for stabilizing nucleic acids for a long time in a wide temperature range (up to 15 days at temperatures ranging from -20°C to +37°C and up to 12 months at temperatures ranging from -86°C to -20°C) using the Medium according to the main aspect of the invention in the Device according to the second aspect of the invention for collecting, storing and transporting biological samples in laboratories with different equipment levels.

The technical result according to the third aspect of the invention is achieved due to the balanced composition of the Medium according to the main aspect of this invention, in which a combination of a barrier gel and a stabilizer located above the barrier gel and containing a universal cell membrane fixing agent that has a stabilizing effect on the cell membrane is implemented, which allows using Devices, mainly vacuum tubes, according to the second aspect of this invention for collecting, storing and transporting biological samples, to storage and transporting samples, mainly a blood sample containing extracellular nucleic acids that need to be preserved, under any conditions and resources available to the laboratory:
- possibility of storage and transportation of the biological specimen without centrifugation for **up to 24 hours** after collection at temperature ranging from +4°C to +37°C with further centrifugation, followed by storage and transportation **from 5 to 15 days,** depending on the stabilizer composition at temperature ranging from -20°C to +37°C;
- possibility of repeated freezing / thawing of the sample with storage at a temperature of -86°C to -20°C **up to 12 months** after centrifugation at any period of storage of a biological specimen from **5 to 15 days,** depending on the stabilizer composition.
- additional possibility of storing and transporting a biological specimen without centrifugation **from 5 to 15 days** after taking, depending on the stabilizer composition at a temperature of +4°C to +37°C with preservation of extracellular nucleic acids, as well as circulating cells and/or extracellular structures, if necessary;
- additional possibility of storing and transporting a biological specimen without centrifugation **from 5 to 15 days** after taking depending on the stabilizer composition at a temperature of +4°C to +37°C, and with centrifugation just before the collection of plasma for removal of cells, extracellular structures, and cellular debris with obtainment of the cell-free plasma.

As a result, it allows implementing the main task of present invention, which is to create a formulation (medium) and a device for collecting, storing and transporting biological samples containing this medium. It would ensure the safety of extracellular nucleic acids for a long time in a wide temperature range, including negative, and at the same time would be suitable for the laboratories with any level of equipment, including medical offices / biomaterial collection points with a minimum equipment level:
- storage and transportation of biological specimen after centrifugation for **up to 15 days** at temperature ranging from -86°C to +37°C with the possibility of repeated freezing/thawing of the specimen, which is relevant for any laboratories during cold season, enables not to depend on strict compliance with certain storage temperature requirements as in case of transportation of frozen plasma specimen at negative temperatures or whole blood specimen at temperature range +(4-37)°C, and to ensure necessary quantity of extracellular nucleic acids in the specimen, thus reducing the probability of repeated blood sampling in patients, of which due to cell hemolysis;
- additional possibility of storage and transportation of biological specimen without centrifugation for **up to 15 days** at temperature ranging from +4°C to +37°C, which is suitable for any laboratory during warm season, but especially for blood collection points / medical offices with limited resources and low equipment level.

The following paragraphs contain terms and definitions related to this invention and are intended to be applied uniformly throughout the specification and claims, except where broader definitions are given. For the purpose of the present invention the following terms are defined:
As used herein, the term "nucleic acid" includes both ribonucleic acid (RNA) and deoxyribonucleic acid (DNA), and also includes RNA and/or DNA that is linear or branched, single or double stranded, or fragments thereof. In particular, the nucleic acids may be extracellular DNA, extracellular RNA, or any combinations thereof. The biological specimen containing the nucleic acids to be preserved may be any biological fluid, and in particular may be blood. More specifically, extracellular nucleic acids can be found in plasma.

In more detail, nucleic acids can be extracellular DNA or RNA from a subject containing its own nucleic acid, and/or a subject to be detected and/or monitored for a cancer, and/or a subject to be detected and/or monitored for a neurodegenerative disease, or a subject to be detected and/or monitored for mental illness, organ transplant rejection, or a subject who is pregnant and requires self- and/or fetal DNA testing, including but not limited to the genetic disease markers, disease susceptibility, gender / rhesus-phenotype / genetic profile / paternity determination during pregnancy.

Biological specimen may also contain without the limitation circulating cells and/or any extracellular structures (including ectosomes and exosomes) of the subject to be detected and/or monitored for oncological disease.

The term "thixotropic barrier gel" means a gel that becomes thinner due to agitation or pressure, i.e., the viscosity of which decreases as a result of agitation or pressure. Therefore, the term "thixotropic barrier gel" used herein refers to a gel-like substance that is thick or viscous under static conditions, but will flow and become less viscous when shaken, agitated, sheared or otherwise stressed ***(***RU2667964C1***).*** The gel creates a reliable barrier between blood cells and plasma, which prevents the release of genomic DNA into plasma during cell hemolysis during sample storage and transportation. In accordance with this invention, the thixotropic gel may be, without limitation, an olefin, polyester, polyamide gel, polyesters, denatured collagens, polypropylenes, polysiloxanes such as dimethylpolysiloxane and ethyltriethoxysilane, hydrocarbon gel materials such as polybutene, and combinations thereof (including but not limited to) may also be used as thixotropic gel components. The thixotropic gel, or mixtures thereof, must be insoluble in water and chemically inert with respect to blood components that can be used in accordance with this invention.

The term "comprising" means including, but not limited to, whatever follows the word "comprising". Thus, use of the term "comprising" indicates that the listed elements are required or mandatory, but that other elements are optional and may or may not be present.

The term "consisting of" means including, and limited to, whatever follows the phrase "consisting of'. Thus, the phrase "consisting of" indicates that the listed elements are required or mandatory, and that no other elements may be present.

### Brief description of the figures

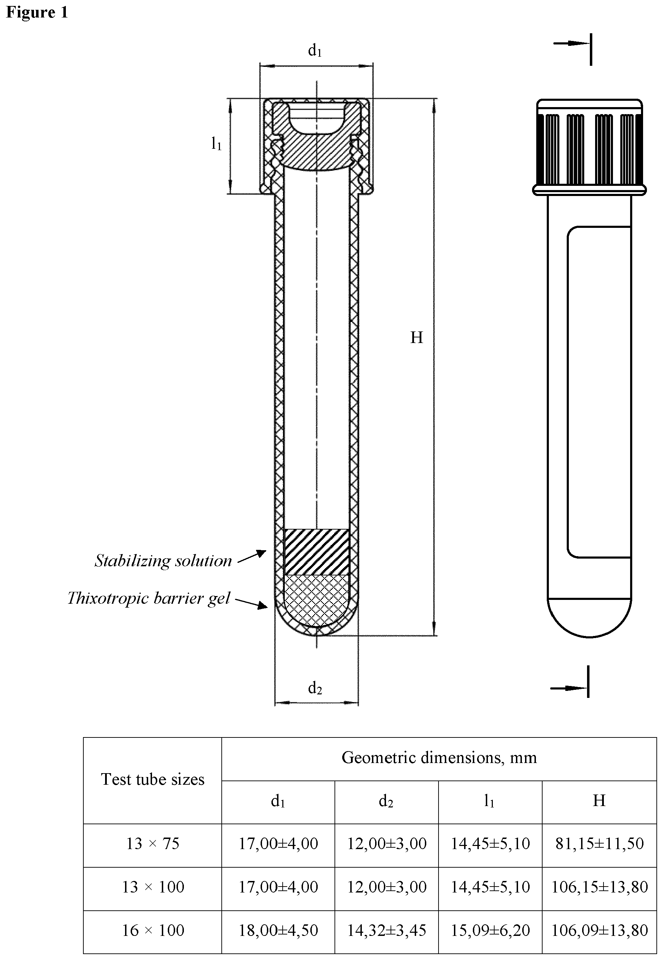

**Figure 1****.** General view of the Device containing the Medium for collecting, storing and transporting biological samples, containing a barrier gel and a stabilizing solution containing at least 1 cell membrane fixing agent.

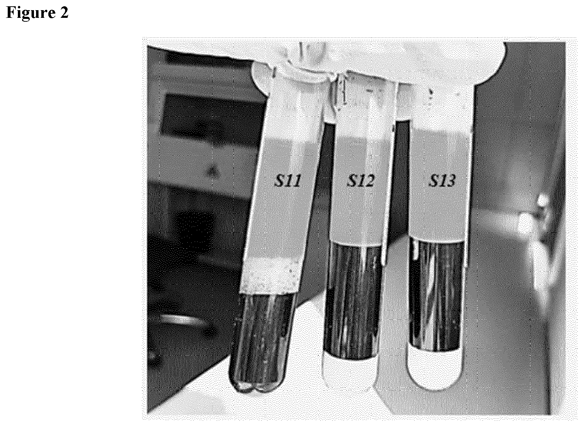

**Figure 2****.** Photographs of tubes with Medium according to the main aspect of the invention and whole blood samples on the 0th day of storage: tube with sample S11 - after centrifugation at room temperature +(15-25)°C for 15 minutes at 3000g; tubes with samples S12 and S13 - without centrifugation.

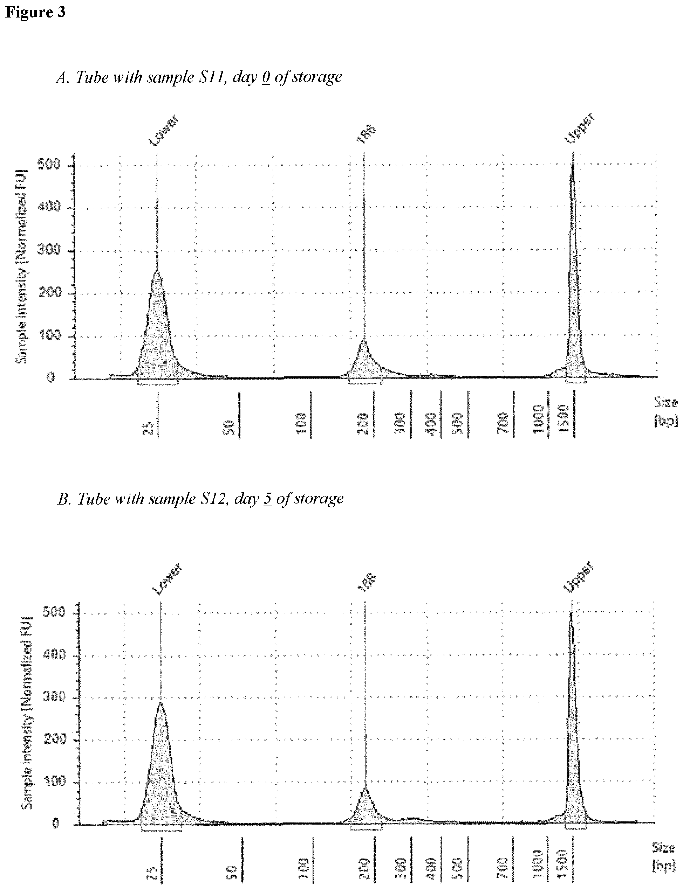

**Figure 3****.** Examples of electropherograms obtained using the TapeStation 2200 automatic capillary electrophoresis system for extracellular DNA samples on days 0 (A) and 5 (B) of storage at room temperature +(15-25)°C after centrifugation immediately before plasma sampling (tubes with samples S11 and S12, respectively).

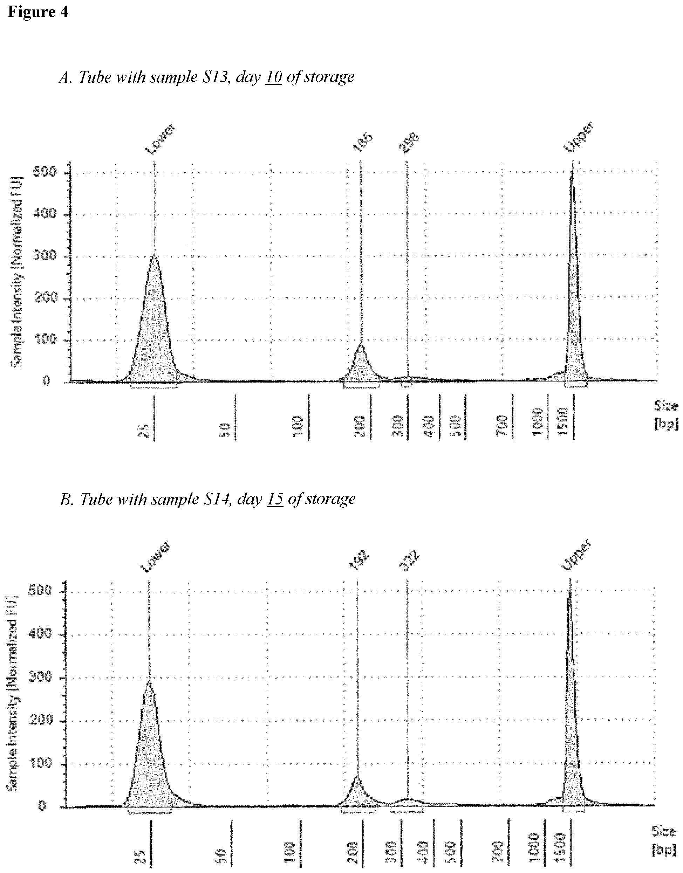

**Figure 4****.** Examples of electropherograms obtained using the TapeStation 2200 automatic capillary electrophoresis system for extracellular DNA samples on day 10 (A) and 15 (B) of storage at room temperature +(15-25)°C after centrifugation immediately before plasma sampling (tubes with samples S13 and S14, respectively).

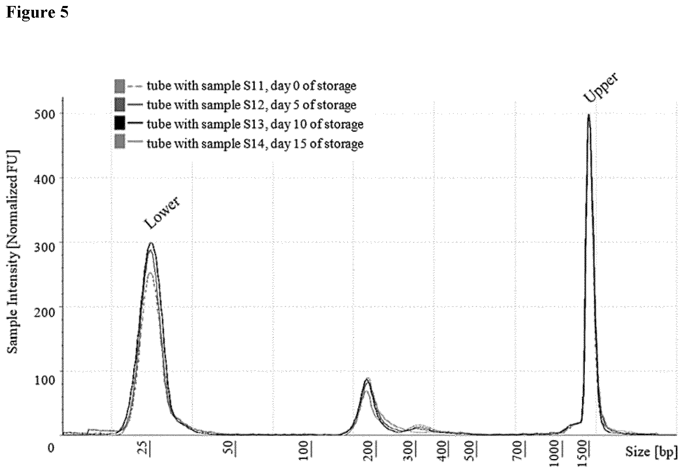

**Figure 5****.** The results of a comparative analysis of the safety of extracellular DNA for samples that were stored without prior centrifugation for day 0, 5, 10 and 15 at room temperature +(15-25)°C and which were subjected to centrifugation immediately before plasma collection (tubes with samples S11, S12 , S13 and S14, respectively).

The task of this invention is to create medium and device for collection, storage and transportation of biological samples containing the said medium, enabling to preserve intracellular nucleic acids during a long time in a wide temperature range and also suitable for use in laboratories with any equipment level, of which for medical offices/ biomaterial sampling points with minimum equipment level.

In turn, the use of the main aspects proposed within the scope of this application, namely the new Medium formulation containing a thixotropic barrier gel and a stabilizer, including at least one cell membrane fixing agent, as well as Device for collecting, storing and transporting biological samples, containing this medium, and methods of their use for the stabilization of nucleic acids can reduce the likelihood that the population of extracellular nucleic acids in a biological specimen will be diluted with intracellular nucleic acids, in particular, fragmented genomic DNA originating from damaged and/or dying (apoptotic) cells, contained in the test sample.

The invention is illustrated by the following particular examples, which confirm the practical possibility of using one of the embodiments of the Medium according to the main aspect of this invention in a Device for collecting, storing and transporting biological samples, to stabilize biological samples, in this case, whole blood samples containing extracellular nucleic acids and cells, during centrifugation at different periods of storage, as well as during transportation and storage in different temperature ranges.

The exemplary embodiment of the Medium contains a stabilizer (0.1 ml per 1 ml of blood) and a thixotropic barrier gel in the Device for collecting, storing and transporting biological samples in the concentration ranges is provided in **Table 1.**

**Table 1. One variant of embodiment of the composition of Medium for collection, storage and transportation of biological samples.**

| *Component* | *Concentration Range, %* |
|---|---|
| Thixotropic barrier gel | 0.3-1.8 (g) |
| Stabilizer | |
| ethylenediaminetetraacetic acid (EDTA) | 0.1-28.3 |
| dimethylol-5,5-dimethylhydantoin | 0.1-28.8 |
| diazolidinylurea | 1.0-34.6 |

*Thixotropic barrier gel* is a special inert barrier gel, which is heavier than serum, but lighter than a blood clot (blood cells), therefore, after centrifugation, the gel in the form of a thin strip occupies an intermediate position and serves as a separating barrier. It allows cells to be isolated from plasma / serum and other biological fluids, which prevents active enzymes from degrading cells from escaping into solution along with additional intracellular DNA and / or RNA, which significantly degrade the quality and reduce the concentration of the primary free-circulating extracellular DNA / RNA present at the moment of taking a biological sample, and also increase the concentration of intracellular DNA and/or RNA.

*Ethylenediaminetetraacetic acid (EDTA)* are chelates (binds) divalent metals in solution (magnesium, calcium, etc.) and thereby inactivates the action of metal-dependent enzymes. It prevents the process of blood coagulation (one of the most common anticoagulants), and also inhibits the work of nucleases, which are metal-dependent, thereby preventing the breakdown / degradation of extracellular nucleic acids.

*Dimethylol-5,5-dimethylhydantoin, diazolidinyl urea* are broad-spectrum preservatives, stabilize the cell membrane through chemical fixation, and also prevent the growth of solutions, as they have activity against a wide range of gram-negative and gram-positive bacteria, molds and fungi. Additionally, **Table 2** provides for the composition of the Medium for the collection, storage and transport of biological specimen in the other three possible embodiments.

**Table 2. Composition of the Medium for the collection, storage and transport of biological specimen in the other three possible embodiments.**

| *Component* | *Concentration Range, %* |
|---|---|
| Thixotropic barrier gel | 0.3-1.8 (g) |
| Stabilizer | |
| sodium citrate | 0.1-28.3 |
| dimethylol urea | 1.0-34.6 |
| 2-bromo-2-nitropropane-1,3 -diol | 0.2-28.8 |
| | |

| *Component* | *Concentration Range, %* |
|---|---|
| Thixotropic barrier gel | 0.3-1.8 (g) |
| Stabilizer | |
| ethylenediaminetetraacetic acid (EDTA) | 0.1-13.1 |
| ethylene glycol tetraacetic acid (EGTA) | 0.1-15.2 |
| 1,3, 5-tris(hydroxyethyl)-s-triazine | 0.1-32.6 |
| 1,3-bis(hydroxymethyl)-5,5-dimethylimidazolidine-2,4-dione | 0.2-30.8 |
| | |

| *Component* | *Concentration Range, %* |
|---|---|
| Thixotropic barrier gel | 0.3-1.8 (g) |
| Stabilizer | |
| ethylenediaminetetraacetic acid (EDTA) | 0.1-28.3 |
| dimethyl-dimethylol-hydantoin | 0.2-63.4 |

However, the examples provided above are for illustrative purposes only and should not be construed as limiting the invention in any way.

According to the present invention, all possible combinations of the composition of the stabilizing solution were analyzed, as described in paragraph 1:

### Cell membrane fixating agent

1. 2-bromo-2-nitropropane-1,3-diol
2. dimethylol urea
3. sodium hydroxymethylglycinate
4. diazolidinyl urea
5. dimethyl-dimethylol-hydantoin
6. imidazolidinyl urea
7. 1,3,5-tris(hydroxyethyl)-s-triazine
8. 1,3-bis(hydroxymethyl)-5,5-dimethylimidazolidine-2,4-dione
9. dimethylol-5,5-dimethylhydantoin
   **anticoagulant/ionic stabilizing agent**
10. ethylene glycol tetraacetic acid (EGTA) and its salts
11. sodium citrate
12. ethylenediaminetetraacetic acid (EDTA) and its salts
13. sodium fluoride

In total, variations in the composition of the stabilizing solution were obtained and analyzed according to **Table 3.**

**Table 3. Combinations of the stabilizer composition analyzed within the framework of present invention.**

| **Component** # | 10 | 10-11 | 10-12 | 10-13 | 11 | 11-12 | 11-13 | 12 | 12-13 | 13 |
|---|---|---|---|---|---|---|---|---|---|---|
| 1 | + | + | + | + | + | + | + | + | + | + |
| 1-2 | + | + | + | + | + | + | + | + | + | + |
| 1-3 | + | + | + | + | + | + | + | + | + | + |
| 1-4 | + | + | + | + | + | + | + | + | + | + |
| 1-5 | + | + | + | + | + | + | + | + | + | + |
| 1-6 | + | + | + | + | + | + | + | + | + | + |
| 1-7 | + | + | + | + | + | + | + | + | + | + |
| 1-8 | + | + | + | + | + | + | + | + | + | + |
| 1-9 | + | + | + | + | + | + | + | + | + | + |
| 2 | + | + | + | + | + | + | + | + | + | + |
| 2-3 | + | + | + | + | + | + | + | + | + | + |
| 2-4 | + | + | + | + | + | + | + | + | + | + |
| 2-5 | + | + | + | + | + | + | + | + | + | + |
| 2-6 | + | + | + | + | + | + | + | + | + | + |
| 2-7 | + | + | + | + | + | + | + | + | + | + |
| 2-8 | + | + | + | + | + | + | + | + | + | + |
| 2-9 | + | + | + | + | + | + | + | + | + | + |
| 3 | + | + | + | + | + | + | + | + | + | + |
| 3-4 | + | + | + | + | + | + | + | + | + | + |
| 3-5 | + | + | + | + | + | + | + | + | + | + |
| 3-6 | + | + | + | + | + | + | + | + | + | + |
| 3-7 | + | + | + | + | + | + | + | + | + | + |
| 3-8 | + | + | + | + | + | + | + | + | + | + |
| 3-9 | + | + | + | + | + | + | + | + | + | + |
| 4 | + | + | + | + | + | + | + | + | + | + |
| 4-5 | + | + | + | + | + | + | + | + | + | + |
| 4-6 | + | + | + | + | + | + | + | + | + | + |
| 4-7 | + | + | + | + | + | + | + | + | + | + |
| 4-8 | + | + | + | + | + | + | + | + | + | + |
| 4-9 | + | + | + | + | + | + | + | + | + | + |
| 5 | + | + | + | + | + | + | + | + | + | + |
| 5-6 | + | + | + | + | + | + | + | + | + | + |
| 5-7 | + | + | + | + | + | + | + | + | + | + |
| 5-8 | + | + | + | + | + | + | + | + | + | + |
| 5-9 | + | + | + | + | + | + | + | + | + | + |
| 6 | + | + | + | + | + | + | + | + | + | + |
| 6-7 | + | + | + | + | + | + | + | + | + | + |
| 6-8 | + | + | + | + | + | + | + | + | + | + |
| 6-9 | + | + | + | + | + | + | + | + | + | + |
| 7 | + | + | + | + | + | + | + | + | + | + |
| 7-8 | + | + | + | + | + | + | + | + | + | + |
| 7-9 | + | + | + | + | + | + | + | + | + | + |
| 8 | + | + | + | + | + | + | + | + | + | + |
| 8-9 | + | + | + | + | + | + | + | + | + | + |
| 9 | + | + | + | + | + | + | + | + | + | + |

The composition according to **Table 1** is selected as a detailed example of implementation of the invention.

For each of the presented embodiments, studies were conducted similar to examples 1 and 2.

In all embodiments, the compliance of all combinations with the required parameters was revealed, where each of the possible combinations ensures the achievement of the stated technical result. For all embodiments, cell-free DNA was demonstrated to be resistant to all tested climatic factors under the specified application conditions and the ability to preserve the sample until analysis. The data obtained for all embodiments according to the invention also indicate the resistance of extracellular RNA to all tested climatic factors in the indicated conditions of use and the ability to save the sample until analysis.

At the same time, the design of the Device for collecting, storing and transporting biological samples according to the second aspect of this invention, in this embodiment of the invention, is a transparent vacuum tube (PET) containing the Medium according to the main aspect of this invention, equipped with a stopper (butyl rubber) and a protective cap (polypropylene). The stabilizer in this embodiment of the invention is located above the barrier gel. The construction in this embodiment corresponds to the drawing in **Figure 1****.**

### Example 1. Stabilization of Extracellular DNA

### Blood Sampling

Blood samples were collected from healthy donors on the basis of informed voluntary consent into the Device for collecting, storing and transporting biological samples according to the second aspect of this invention, which in this embodiment is a vacuum tube containing the Medium according to the main aspect of this invention, having the composition as indicated in **Table 1.** Within the scope of this experiment, samples were intended for:
- checking the possibility of storing and transporting the sample without centrifugation **for 8 hours** after blood sampling at a temperature ranging from +4°C to +37°C, followed by centrifugation and then storage and transportation for 15 days at a temperature ranging from -20°C to +37°C;
- checking the possibility of triple freezing / thawing of the sample after centrifugation at any period of storage of the sample **within 15 days** with storage at a temperature ranging from -86°C to -20°C **for 6 months**

To solve these problems, the following blood samples were taken:
∘ *2 standard size 16*×*100 test tubes from each donor for further storage at room temperature* +*(15-25)°C after centrifugation* - checked on days 0, 5, 10, 15 with triple freezing / thawing of tubes with samples at a temperature of -86 °C - samples *S1, S2;*
∘ *2 standard size 16*×*100 test tubes from each donor for further storage in a dry-air thermostat at a temperature of* + *37°C after centrifugation* - checked on days 0, 5, 10, 15 with triple freezing / thawing of tubes with samples at a temperature of -86 °C - samples *S3, S4;*
o *2 standard size 16*×*100 test tubes from each donor for further storage in fridge at temperature* +*(4-8)°C after centrifugation* - checked on days 0, 5, 10, 15 with triple freezing / thawing of tubes with samples at a temperature of -86 °C - samples *S5, S6;*
o *2 standard size 16*×*100 test tubes from each donor for further storage in a freezer at temperature -20°C after centrifugation* - checked on days 0, 5, 10, 15 - samples *S7, S8.*
   - checking the additional possibility of storing and transporting a biological specimen without centrifugation **from 5 to 15 days** after blood sampling, depending on the combination of components of the stabilizer at a temperature of +4°C to +37°C, while preserving extracellular nucleic acids, as well as circulating cells and/or extracellular structures, if necessary.

To solve these problems, the following blood samples were taken:
∘ *2 standard size 16*×*100 test tubes from each donor for further storage at room temperature without centrifugation* - checked on days 0, 5, 10, 15 - samples *S9, S10* (safety of cells and extracellular structures was assessed visually by the presence of hemolysis at different periods of storage).
   - checking the additional possibility of storing and transporting a biological specimen without centrifugation **from 5 to 15 days** after blood sampling, depending on the combination of components of the stabilizer solution at a temperature of +4°C to +37°C, and with centrifugation immediately before plasma collection to remove cells, extracellular structures and cellular debris to obtain cell-free plasma.

To solve these problems, the following blood samples were taken:
o *4 standard size 16*×*100 test tubes from each donor for further storage at room temperature with centrifugation immediately before plasma collection* - checked on days 0, 5, 10, 15 - samples *S11-S14.*

Blood samples were taken into vacuum tubes with Medium according to the main aspect of the invention by way of venipuncture with standard double-ended needles. The vacuum tube was filled to the mark (black square on the label of vacuum tube). The tube was then removed from the needle holder/adapter and immediately mixed gently by inverting the tube 10 times (without shaking). One turn of the test tube is a complete rotation of the wrist 180 degrees and back.

### Preparation of plasma

### Samples S1-S8:

**8 hours** after taking venous blood, the tubes with samples *S1-S8* were centrifuged at room temperature +(15-25)°C for 15 minutes at 3000g, and then placed for storage under different temperature conditions:
- at room temperature: 0 and 5^{th} day - sample *S1,* 10^{th} and 15^{th} day - sample *S2*;
- in a dry-air thermostat at a temperature +37°C: 0 and 5^{th} day - sample *S3*, 10^{th} and 15^{th} day - sample *S4;*
- in a refrigerator at temperature +(4-8)°C: 0 and 5^{th} day - sample *S5*, 10^{th} and 15^{th} day - sample *S6;*
- in freezer at temperature -20°C: 0 and 5^{th} day - sample *S7*, 10^{th} and 15^{th} day - sample *S8.*

The selection and preparation of plasma for the study was carried out on days 0, 5, 10 and 15 of storage as follows:
- ½ of the plasma volume was taken into a clean Eppendorf-type test tube, if the total volume of ≈ 3-3.5 ml of plasma was in the test tubes with a thixotropic barrier gel before testing at the analyzed storage period *(if there was already ½ of the plasma volume in the test tube, then the entire remaining volume);*
- centrifugation of the analyzed sample at temperature +4°C within 10 minutes at 16000g;
- careful separation of the supernatant and transfer to a clean Eppendorf tube;
- extraction of extracellular DNA from 1.5 ml of plasma according to the manufacturer instructions.

The following was additionally carried out in the case of tubes with sample S7 and S8 stored at - 20°C, as well as in the analysis of samples from tubes frozen at -86°C:
- thawing of tube at room temperature;
- gentle mixing 4-6 times or gentle pipetting;
   > further, similarly to test tubes *S1-S6,* plasma was collected and prepared, followed by extraction of extracellular DNA according to the manufacturer's instructions;
- repeated freezing of tube with remaining ½ volume of plasma, gel, and blood cells, if necessary.

### Samples S9, S10:

Stabilized blood samples were stored at room temperature in an upright position. The selection and preparation of plasma for the study was carried out on days 0.5 (tube with sample S9) and 10.15 days of storage (tube with sample *S10*) as follows:
- gentle mixing 6-8 times and subsequent sampling of ½ of the blood volume into an Acti-Fine^{®} vacuum tube without filler produced by Granat Bio Tech (SEZ Dubna, Russia), if the tube had a full volume of blood before testing at the planned storage period ≈ 7.5 ml (*if there was already ½ volume of whole blood in the tube, then gently mixing 6-8 times and transferring the entire remaining volume to the Acti-Fine^{®} tube without filler);*
- centrifugation of the selected blood sample in test tubes at room temperature for 15 minutes at 1600g;
- careful separation of the supernatant, followed by transfer to a clean Eppendorf tube and repeated centrifugation to separate the cellular debris at +4°C for 10 minutes at 16000g;
- careful separation of the supernatant, transferring it to a clean Eppendorf tube;
- extraction of extracellular DNA according to the manufacturer instructions.

### Samples S11-S14:

Stabilized blood samples were stored at room temperature in an upright position. Extraction and preparation of plasma for the study was carried out on 0, 5^{th}, 10^{th} and 15 day (tubes with sample *S11, S12, S13 and S14)* as follows:
- centrifugation of tubes immediately before plasma isolation at room temperature +(15-25)°C during 15 minutes at 3000g;
- 1.5-2 ml of plasma were taken into a clean Eppendorf tube;
- centrifugation of the analyzed sample in tubes at temperature +4°C during 10 minutes at 16000g;
- careful separation of the supernatant, transferring it to a clean Eppendorf tube;
- extraction of extracellular DNA according to the manufacturer instructions.

Examples of photographs of test tubes with the medium according to the main aspect of this invention before and after centrifugation are shown in **Figure 2****.**

### Extraction of Extracellular DNA

Extracellular DNA was extracted from plasma with the use of "MagMAX^{™} Cell-Free DNA Isolation Kit" (ThermoFisher Scientific, USA). Extraction was carried out according to protocol No. 2 without addition of protein kinase. The kit includes buffer solutions and magnetic particles with which extracellular DNA molecules bind. At the first stage, a plasma sample in a volume of 1.5 ml was placed in 15 ml Falcon-type tubes.

2.53 ml of a pre-prepared solution containing the LysingBinding buffer and magnetic particles, which were preliminarily mixed in a Microspin FV-2400 mini-vortex centrifuge (Biosan, Latvia), was added to each tube. The tubes were carefully inverted 10 times, and then all the tubes were vigorously shaken on a vortex for 10 minutes (this step is necessary for the binding of extracellular DNA to magnetic particles). Next, the tubes were placed incubated in a magnetic rack for 5 minutes, and then the clear supernatant was removed, while the magnetic particles remained on the walls of the tubes.

The next steps included washing the magnetic particles with a special wash buffer (MagMAX^{™} Cell Free DNA Wash Solution). To do this, 1 ml of buffer was added to the magnetic particles and the contents of the tubes were mixed on a vortex for 30 seconds. The resulting solution containing wash buffer and magnetic particles was transferred into 2 ml Eppendorf tubes.

These tubes were placed on a magnetic rack for 2 ml tubes and then left for 3 minutes until the buffer became transparent and the magnetic particles were completely fixed on the walls of the tube. The buffer was then aspirated.

The second washing step included a repetition of the first one: 1 ml of washing buffer was added to the magnetic particles -> the contents were mixed on a vortex for 30 seconds -> the tubes were returned to the magnetic rack -> the buffer was aspirated.

The next 2 wash steps involved using freshly made 70% ethanol. To do this, 1 ml of 70% ethanol was added to 2 ml Eppendorf type tubes -> the contents were mixed on a vortex for 30 seconds - > the tubes were returned to the magnetic stand -> ethanol was aspirated -> this stage was performed 2 times.

Next, the magnetic particles were dried from residual ethanol for 2-3 minutes and 15 µl of extracellular DNA elution buffer was added. At the same time, for efficient transfer of DNA into the buffer solution, after adding the elution buffer to the magnetic particles, the contents of the tubes were pipetted and left in a thermal shaker for microtubes and PCR plates TS-100 (Biosan, Latvia) at maximum speed for five minutes. Next, the tubes were again placed in a magnetic rack until the liquid became transparent and all magnetic particles were collected on the side walls of the tubes. The liquid containing the eluted DNA was carefully aspirated and transferred to a new 0.5 ml Eppendorf tube (DNA LoBind).

### Assessment of the stabilizing effect of one of the embodiments of the Medium for collection, storage and transport of biological samples using the Tape Station 2200

To assess the effectiveness of the stabilizing effect of one of the embodiments of the Medium (according to **Table 1**) on the main aspect of the invention in the Device for collecting, storing and transporting biological samples, namely the ability to stabilize extracellular nucleic acids and cells of a biological sample, in this case a whole blood sample, a qualitative determination of the presence of extracellular DNA in tubes with samples S1-S14 from different donors without/with centrifugation at different periods of storage, as well as during transportation and storage in different temperature ranges, was carried out. Qualitative determination of the presence of extracellular DNA was performed by capillary electrophoresis on TapeStation 2200 equipment (Agilent, USA) using ready-made reagent kits in accordance with the manufacturer's instructions. The determination of extracellular DNA was carried out by the presence of characteristic fluorescence peaks on the electropherogram, while the fluorescent peaks had to meet the requirements of **Table 4.**

**Table 4. Requirements for extracellular DNA obtained from whole blood samples before and/or after centrifugation at different periods of storage, as well as during transportation and storage in different temperature ranges.**

| Characteristic | Day of comparison during the storage period of the blood sample | | | |
|---|---|---|---|---|
| | 0 | 5 | 10 | 15 |
| The presence of a fluorescence peak in the range of fragment lengths of 150-250 bps (pairs of nucleotides) characteristic of extracellular DNA | + | + | + | + |
| Change in the intensity (height) of the fluorescence peak characteristic of extracellular DNA at different storage periods relative to day 0 of storage (the day of venous blood sampling into vacuum tubes by venipuncture - day 0 of comparison) | - | from 0.25 to 4 times | from 0.25 to 4 times | from 0.25 to 4 times |

In turn, the assessment of the presence and determination of the length of fragments of extracellular DNA separated from plasma by capillary electrophoresis was performed using the D1000 ScreenTape System kit and preliminary sample preparation according to the following procedure. 2 µl of buffer *(D1000 Sample Buffer*) was added to each 0.2 ml tube in strip format, consisting of 8 tubes with separately attached striped flat caps. Next, 2 µl of a DNA fragment length marker (*D1000 Sample Ladder)* was added to the first tube, and extracellular DNA samples were added to the remaining tubes in an amount of approximately 0.5-1 ng per well, while the sample volume was 2 µl. The concentration of extracellular DNA was preliminarily measured using a Qubit 4.0 fluorimeter (ThermoFisher Scientific, USA) using a High Sensitivity dsDNA kit according to the protocol recommended by the manufacturer. The total volume of the contents of each tube was 4 µl. Test tubes in strips of 2 pieces were loaded into the automatic TapeStation 2200 system together with dosing tips and a special chip for capillary electrophoresis, and then the program was launched to obtain and analyze the results. Examples of results obtained using the TapeStation 2200 are shown in **Figures 3-5****.**

Additional examples of test results of one of the embodiments of the Medium according to the main aspect of the invention in the Device for collecting, storing and transporting biological samples, for its ability to stabilize extracellular DNA in whole blood samples without/with centrifugation at different storage periods in different temperature ranges are given in **Tables 5 and 6.**

As a result of the testing of one of the embodiments of the Medium according to the main aspect of the invention, containing a stabilizer and a thixotropic barrier gel in the Device for collecting, storing and transporting biological samples in the concentration ranges indicated in **Table 1,** the resistance of extracellular DNA to the effects of all tested climatic conditions under specified conditions of use and the ability to preserve the sample prior to analysis was demonstrated.

For all embodiment variants of the medium, similar tests were carried out. In all variants of the compositions of the medium, the resistance of extracellular DNA to the effects of all tested climatic factors under the indicated conditions of use and the ability to save the sample prior to analysis was demonstrated.

In the case of extracellular DNA, all given combinations were checked for compliance with the parameters indicated in **Table 4.**

**Table 5. Results of checking the safety of extracellular DNA in samples that were subjected to centrifugation for 8 hours after blood sampling and then stored for 15 days at temperatures of +4°C, +(15-25)°C and +37°C with triple freezing / thawing of samples at temperature -86°C.**

| **Donor #** | **Requirement of Table 4** | | **The compared day during the period of storage of the blood sample at temperature:** | | | | | | Conclusion |
|---|---|---|---|---|---|---|---|---|---|
| | | | **+4°C** | | **+(15-25)°C** | | **+37°C** | | |
| | Characteristic | | 0 *(S1)* | 15 *(S2)* | 0 *(S3)* | 15 *(S4)* | 0 *(S5)* | 15 *(S6)* | |
| 1 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0,94 | - | 0,91 | - | 0,83 | Complies |
| 2 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0,87 | - | 0,84 | - | 0,76 | Complies |
| 3 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0.95 | - | 0,88 | - | 0,75 | Complies |
| 4 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 1,00 | - | 0,82 | - | 0,87 | Complies |
| 5 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0,90 | - | 0,89 | - | 0,85 | Complies |

**Table 6. Results of checking the safety of extracellular DNA in samples that were subjected to centrifugation for 8 hours after blood sampling and then stored for 15 days at a temperature of - 20°C, as well as samples that were stored for 15 days after blood sampling blood at room temperature +(15-25)°C without centrifugation or with centrifugation immediately before plasma sampling.**

| Donor # | Requirement of **Table 4** | | Day of comparison during the period of storage of the blood sample: | | | | | | Conclusion |
|---|---|---|---|---|---|---|---|---|---|
| | | | Blood sample storage temperature: -20°C | | Blood sample storage temperature: +(**15-25**)°C | | | | |
| | | | | | Without centrifugation | | with centrifugation immediately before taking plasma | | |
| | Characteristic | | 0 *(S7)* | 15 *(S8)* | 0 *(S9)* | 15 *(S10)* | 0 *(S11)* | 15 *(S14)* | |
| 6 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0,97 | - | 0,86 | - | 0,85 | Complies |
| 7 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 1.03 | - | 0.93 | - | 0.92 | Complies |
| 8 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 025 to 4 times | - | 0,99 | - | 0,92 | - | 0,90 | Complies |
| 9 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0,96 | - | 0,91 | - | 0,92 | Complies |
| 10 | The presence of a fluorescence peak in the specified range | 150-250 bps | + | + | + | + | + | + | Complies |
| | Change in the intensity (height) of the fluorescence peak characteristic of extracellular DNA at different storage periods relative to day 0 of storage (the day of venous blood sampling into vacuum tubes by venipuncture - day 0 of comparison) | from 0.25 to 4 times | - | 0,95 | - | 0,88 | - | 0,84 | Complies |

### Example 2. Stabilization of Extracellular RNA

### Blood Sampling

Blood samples were taken from healthy donors on the basis of informed voluntary consent in the Device for collecting, storing and transporting biological samples according to the second aspect of the invention, which in this embodiment is a vacuum tube containing the Medium according to the main aspect of the invention, having a composition according to **Table 1.**

Within the scope of this study samples were intended for:
- checking the possibility of storage and transportation of sample without centrifugation within **8 hours** after blood sampling at temperature ranging from +4°C to +37°C with further centrifugation and storage and transportation within **15 days** at temperature ranging from -20°C to +37°C;
- checking the possibility of repeated freezing/ thawing of sample after centrifugation at any term of storage within **15 days** with storage at temperature ranging from -86°C to -20°C within **6 months.**

For solution of these tasks the following samples were collected:
∘ *2 standard size 16×100 test tubes from each donor for further storage at room temperature* +*(15-25)°C after centrifugation* - checked on days 0, 4, 7, 10, 15 with triple freezing/ thawing of tubes with samples at temperature -86°C - samples *S15, S16;*
∘ *2 standard size 16*×*100 test tubes from each donor for further storage in dry air thermostat at temperature +37°C after centrifugation* - checked on days 0, 4, 7, 10, 15 with triple freezing/ thawing of tubes with samples at temperature -86°C - samples *S17, S18;*
∘ *2 standard size 16*×*100 test tubes from each donor for further storage in refrigerator at temperature* +*(4-8)°C after centrifugation* - checked on days 0, 4, 7, 10, 15 with triple freezing/ thawing of tubes with samples at temperature -86°C - samples *S19, S20;*
∘ *2 standard size 16*×*100 test tubes from each donor for further storage in freezer at temperature -20°C after centrifugation* - checked on days 0, 4, 7, 10, 15 - samples *S21, S22;*
   - checking the additional possibility of storage and transportation of biological specimen without centrifugation for **5 to 15 days** after sampling depending on the composition of the stabilizer at temperature ranging from +4°C to +37°C with preservation of extracellular nucleic acids, as well as circulating cells and /or extracellular structures, if necessary;

For solution of these tasks the following samples were collected:
∘ *2 standard size 16*×*100 test tubes from each donor for further storage at room temperature without centrifugation* - checked on days 0, 4, 7, 10, 15 - samples S23, *S24* (the safety of cells and extracellular structures was assessed visually by the presence of hemolysis at different periods of storage).
   - checking the additional possibility of storage and transportation of biological specimen without centrifugation for **5 to 15 days** after sampling depending on the composition of the stabilizer at temperature ranging from +4°C to +37°C and centrifugation immediately before collection of plasma for removal of cells, extracellular structures, and cell debris with obtainment of cell free plasma.

For solution of these tasks the following samples were collected:
∘ *4 standard size 16*×*100 test tubes from each donor for further storage at room temperature with centrifugation immediately before extraction of plasma* - checked on days 0, 4, 7, 10, 15 - samples *S25-S29.*

Blood samples were taken into vacuum tubes with Medium according to the main aspect of the invention in the composition according to **Table 1** by way of venipuncture with standard double-ended needles. The vacuum tube was filled to the mark (black square on the label of the vacuum tube). The tube was then removed from the needle holder/adapter and immediately mixed by gently inverting the tube 10 times (without shaking). One turn of the test tube is a complete rotation of the wrist 180 degrees and back.

Additionally, a blood sample was taken from each donor in BD Vacutainer^{®} Plus vacuum tubes with EDTA as an additional control on day 0 of storage. Tubes with EDTA and biological samples were kept on ice and all manipulations were carried out within 1 hour after blood sampling. Fractionation of whole blood in tubes with EDTA, namely, obtaining plasma free of cellular elements, was carried out by centrifugation at room temperature for 20 minutes at low speed (900g), since centrifugation of blood at high speed inevitably leads to the destruction of some of the cells and the entry of cells contained in of them nucleic acids into plasma. Plasma free of formed elements was carefully collected and immediately used for RNA separation. In turn, vacuum tubes with the Medium according to the main aspect of the invention and the biological sample were subjected to centrifugation at higher speeds (3000g) due to the presence of a cell membrane fixing agent in the stabilizing solution, which prevents the destruction of cells and the release of intracellular RNA.

### Preparation of Plasma

### Samples S15-S22:

**8 hours** after venous blood sampling, tubes with samples S15, S22 were centrifuged at room temperature +(15-25)°C for 15 minutes at 3000g, followed by storage of the tubes under different temperature conditions:
- at room temperature: day 0, 4, 7 - samples *S15*; day 10, 15 - sample *S16;*
- in dry-air thermostat at 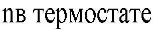 temperature +37°C: days 0, 4, 7 - sample *S17;* day 10, 15 - day *S18*;
- in refrigerator at temperature +(4-8)°C: days 0, 4, 7 - sample *S19*; days 10, 15 - sample *S20;*
- in freezer at temperature -20°C: days 0, 4, 7 - sample S21; day 10, 15 - sample *S22.*

The selection and preparation of plasma for the study was carried out on days 0, 4, 7, 10 and 15 of storage as follows:
- 1 ml of plasma was collected into a clean Eppendorf tube;
- analyzed sample was centrifuged at +4°C for 10 minutes at 16000g;
- careful separation of the supernatant, its transfer to a clean Eppendorf tube;
- extracellular RNA was isolated according to the manufacturer's instructions.

In the case of tubes S21 and *S22* stored at -20°C, as well as in the analysis of samples from tubes frozen at -86°C, additionally given:
- thawing of tube at room temperature;
- gentle mixing 4-6 time or soft pippeting;
   > further, similarly to test tubes with samples *S15-S20,* plasma was collected and prepared, followed by isolation of extracellular RNA according to the manufacturer's instructions;
- re-freezing of the tube with the remaining ½ volume of plasma, gel and blood cells, if necessary.

### Samples S23, S24:

Stabilized blood samples were stored at room temperature in an upright position. The selection and preparation of plasma for the study was carried out on days 0, 4, 7 (tube with sample *S23*) and 10, 15 days of storage (tube with sample *S24*) as follows:
- gentle mixing 6-8 times and subsequent sampling of 2.5 ml of blood into an Acti-Fine^{®} vacuum tube without filler manufactured by Granat Bio Tech (SEZ Dubna, Russia);
- centrifugation of the selected blood sample in test tubes at room temperature for 15 minutes at 1800g;
- the supernatant was carefully separated, transferred to a clean Eppendorf tube and recentrifuged to separate the cellular debris at +4°C for 10 minutes at 16000g;
- carefully separation of the supernatant, its transfer to a clean Eppendorf tube;
- extracellular RNA was separated according to the manufacturer's instructions.

### Samples S25-S29:

Stabilized blood samples were stored at room temperature in an upright position. The selection and preparation of plasma for the study was carried out on days 0, 4, 7, 10 and 15 of storage (tubes *S25, S26, S27, S28 and S29,* respectively) as follows:
- immediately before plasma sampling, the tubes were centrifuged at room temperature +(15-25)°C for 15 minutes at 3000g;
- 1 ml of plasma was taken into a clean Eppendorf tube;
- centrifugation of the selected blood sample in test tubes at temperature +4°C for 10 minutes at 16000g;
- carefully separation of the supernatant, its transfer to a clean Eppendorf tube;
- extracellular RNA was isolated according to the manufacturer's instructions.

### Extraction of RNA

Extraction of RNA from plasma for further staging of the reverse transcription reaction was carried out using the Proba-NK kit (OOO DNA-Technology, Russia) in triplicate. 300 µl of the lysing solution were added to pre-marked Eppendorf-type tubes with a volume of 1.5 ml, without touching the edge of the tube. Next, 100 µl of the studied plasma samples were added to the test tubes, the caps of the tubes were tightly closed and shaken on a vortex for 3-5 sec. Next, the tubes were thermostated at a temperature of 65°C for 15 minutes and the condensate was precipitated by centrifugation at 13,000 rpm for 30 seconds at room temperature.

After that, 400 µl of the precipitation reagent was added to each tube, vortexed for 3-5 seconds, and the tubes were centrifuged at 13,000 rpm for 15 minutes at room temperature. Without touching the sediment, the supernatant was completely removed from each tube with a separate tip, 500 µl of wash solution No. 1 was added to the sediment, the caps of the tubes were closed, and the tubes were carefully inverted 3-5 times. Next, the tubes were centrifuged at 13,000 rpm for 5 min at room temperature. Without touching the sediment, the supernatant was completely removed from each tube with a separate tip, 300 µl of washing solution No. 2 was added to the sediment, and the caps of the tubes were closed and mixed, gently inverting the tubes 3-5 times. Next, the tubes were centrifuged at 13,000 rpm for 5 min at room temperature. Without touching the precipitate, the supernatant was completely removed from each tube with a separate tip and the precipitate was dried at a temperature of 65°C for 5 min in tubes with open lids.

After that, 16.5 µl of dissolution buffer was added to the precipitate, the tubes were shaken on a vortex for 3-5 sec, and drops were precipitated by centrifugation of the tubes for 3-5 sec. The resulting RNA preparations were immediately used to set up the reverse transcription reaction.

### Setting up a reverse transcription reaction (obtaining cDNA)

In the course of reverse transcription of RNA, cDNA was obtained with significantly greater stability, which made it possible to store samples in the future or to analyze them without significant degradation of nucleic acids for a long time.

To carry out the reverse transcription reaction, 0.5 ml Eppendorf tubes were labeled, and the contents of the "RT buffer" and "RT-RANDOM + dNTP primers" tubes from the kit of reagents for reverse transcription (OOO DNA-Technology, Russia) were thawed at room temperature, then the tubes were shaken for 3-5 sec and centrifuged at 1000 rpm for 3-5 sec on a Microspin FV-2400 microcentrifuge-vortex (Biosan, Latvia). In a separate tube, an RT mixture was prepared for setting up the reverse transcription reaction by mixing 2.0 × (N + 1) µl of RT buffer, 1.0 × (N + 1) µl of primers RT RANDOM + dNTP, 0.5 × (N +1) µl of reverse transcriptase, where N is the number of analyzed samples (N) with a margin for 1 sample. Next, 3.5 µl of the RT-mixture was added to the pre-labeled tubes, 16.5 µl of isolated RNA samples were added, shaken on a vortex for 3-5 sec, and drops were precipitated by centrifugation at 1000 rpm for 3-5 sec.

After that, the tubes were placed in a thermostat and incubated at a temperature of +40°C for 30 minutes, then at a temperature of +95°C for 5 minutes. Next, to precipitate the condensate, centrifugation was performed at 13,000 rpm for 30 seconds, and 35 µl of the dissolution buffer from the nucleic acid isolation kit were added to the resulting cDNA. The tubes were shaken on a vortex for 3-5 sec and drops were precipitated by centrifugation at 1000 rpm for 3-5 sec. The resulting cDNA samples were used as a template for real-time polymerase chain reaction (PCR).

### Setting up real-time PCR

The cDNA samples obtained as a result of reverse transcription were used to assess the level of expression of the housekeeping gene, beta-2-microglobulin (b2M), by real-time PCR. Real-time quantitative PCR was performed using a ready-made iQ SYBR Green Supermix PCR system (Bio-Rad, USA). The reaction mixture for PCR with a total volume of 25 µl contained 12.5 µl of twofold iQ SYBR Green Supermix (Bio-Rad, USA), 5 pmol of forward (5'-CGTACTCCAAAGATTCAGGTT-3') and reverse (5'TGCTGCTTACATGTCTCGAT-3') primers to the regions present in the b2M gene transcript, the sequences and optimal amplification conditions of which were obtained from the literature data [8], 5 µl of water without nucleases (Thermo Scientific, USA) and 5 µl of cDNA per reaction. PCR was performed using a DT-Prime amplifier (OOO DNA Technology, Russia) under the following conditions: 1) 95°C, 5 min; 2) 90°C, 10 s; 3) 64°C, 15 s; 4) 72°C, 20 s (45 cycles 2-->4). Fluorescence detection was performed at 72°C at the end of each cycle. Fluorescent signal accumulation curves were analyzed using the FAM channel. For each sample, three technical replicates of the PCR were performed, with the results obtained representing the average threshold cycle (Ct) of the passage of the reaction with certain standard deviations (SD). The analysis of the obtained results was carried out using the software of the cycler used. Additionally, the value of ΔCt = CtEDTA (average threshold cycle of the cDNA sample obtained using vacuum tubes BD Vacutainer^{®} Plus with EDTA on day 0 of storage) - Ct sample (average threshold cycle of the studied cDNA sample obtained using one of the embodiments of the Medium according to the main aspect of the invention in the Device for collecting, storing and transporting biological samples, at different periods of storage at different temperature conditions). Based on the results of evaluating the average values of b2M gene expression, a conclusion was made about the safety of samples of extracellular RNA.

Examples of test results of one of the embodiments of the Medium according to the main aspect of the invention according to **Table 1** in the Device for collecting, storing and transporting biological samples, for its ability to stabilize extracellular RNA in whole blood samples without / with centrifugation at different storage periods in different temperature ranges are given in **Tables 7 and 8.**

**Table 7. The results of checking the safety of extracellular RNA for samples that were subjected to centrifugation 8 hours after blood sampling and then stored for 15 days at temperatures of +4°C, +(15-25)°C and +37°C with triple freezing / thawing of samples at temperature -86°C.**

| Donor | Characteristic | Day 0 (EDTA) | **Compared day during the period of storage of the blood sample at the sample storage temperature:** | | | | | | | | |
|---|---|---|---|---|---|---|---|---|---|---|---|
| | | | **+4°C** | | | **+(15-25)°C** | | | **+37°C** | | |
| | | | 0 *(S15)* | 7 *(S15)* | 15 *(S16)* | 0 *(S17)* | 7 *(S17)* | 15 *(S18)* | 0 *(S19)* | 7 *(S19)* | 15 *(S20)* |
| 1 | Ct | 37.9 | 28.6 | 29.8 | 32.5 | 28.4 | 30.1 | 33.7 | 28.5 | 30.5 | 34.5 |
| | SD | 0.3 | 0.3 | 0.4 | 0.3 | 0.4 | 0.4 | 0.5 | 0.2 | 0.3 | 0.5 |
| | ΔCt | 0.0 | 9.3 | 8.1 | 4.4 | 9.5 | 7.8 | 4.2 | 9.4 | 7.3 | 3.4 |
| 2 | Ct | 37.2 | 28.4 | 30.1 | 32.8 | 28.6 | 30.5 | 33.2 | 28.3 | 31.0 | 33.9 |
| | SD | 0.4 | 0.3 | 0.5 | 0.4 | 0.3 | 0.3 | 0.5 | 0.3 | 0.4 | 0.5 |
| | ΔCt | 0.0 | 8.8 | 7.1 | 4.4 | 8.6 | 6.7 | 4 | 8.9 | 6.2 | 3.3 |
| 3 | Ct | 36.8 | 27.9 | 29.6 | 32.4 | 28.3 | 30.1 | 32.6 | 28.0 | 30.8 | 33.2 |
| | SD | 0.3 | 0.3 | 0.4 | 0.4 | 0.3 | 0.3 | 0.4 | 0.4 | 0. 5 | 0.5 |
| | ΔCt | 0.0 | 8.9 | 7.2 | 4.4 | 8.5 | 6.7 | 4.2 | 8.8 | 6 | 3.6 |
| 4 | Ct | 37.0 | 28.2 | 29.9 | 32.2 | 28.6 | 30.5 | 32.9 | 28.3 | 30.8 | 33.8 |
| | SD | 0.4 | 0.5 | 0.3 | 0.4 | 0.3 | 0.3 | 0.3 | 0.5 | 0.4 | 0.5 |
| | ΔCt | 0.0 | 8.8 | 7.1 | 4.8 | 8.4 | 6.5 | 4.1 | 8.7 | 6.2 | 3.2 |
| 5 | Ct | 37.4 | 28.0 | 30.1 | 32.5 | 27.8 | 29.8 | 32.7 | 28.5 | 30.8 | 33.4 |
| | SD | 0.4 | 0.3 | 0.4 | 0.3 | 0.5 | 0.4 | 0.4 | 0.3 | 0.3 | 0.4 |
| | ΔCt | 0.0 | 9.4 | 7.3 | 4.9 | 9.6 | 7.6 | 4.7 | 8.9 | 6.5 | 4 |

**Table 8. Results of checking the safety of extracellular RNA in samples that were subjected to centrifugation 8 hours after blood sampling and then stored for 15 days at a temperature of -20°C, as well as samples that were stored for 15 days after blood sampling blood at room temperature +(15-25)°C without centrifugation or with centrifugation immediately before plasma sampling.**

| Donor # | Characteristic | Day 0 (EDTA) | Compared day during the period of blood sample storage | | | | | | | | |
|---|---|---|---|---|---|---|---|---|---|---|---|
| | | | Blood sample storage temperature: -20°C | | | Blood sample storage temperature: +(15-25)°C | | | | | |
| | | | | | | Without centrifugation | | | With centrifugation immediately before plasma collection | | |
| | | | 0 *(S21)* | 7 *(S21)* | 15 *(S22)* | 0 *(S23)* | 7 *(S23)* | 15 *(S24)* | 0 *(S25)* | 7 *(S27)* | 15 *(S29)* |
| 6 | Ct | 36.9 | 28.3 | 28.9 | 29.2 | 31.8 | 32.2 | 34.5 | 31.4 | 32.4 | 34.2 |
| | SD | 0.3 | 0.3 | 0.4 | 0.4 | 0.3 | 0.5 | 0.3 | 0.3 | 0.4 | 0.5 |
| | ΔCt | 0.0 | 8.6 | 8 | 7.7 | 5.1 | 4.7 | 2.4 | 5.5 | 4.5 | 2.7 |
| 7 | Ct | 37.2 | 28.8 | 28.6 | 29.3 | 31.8 | 32.5 | 35.2 | 31.8 | 32.8 | 35.1 |
| | SD | 0.4 | 0.2 | 0.3 | 0.3 | 0.5 | 0.3 | 0.4 | 0.3 | 0.4 | 0.4 |
| | ΔCt | 0.0 | 8.4 | 8.6 | 7.9 | 5.4 | 4.7 | 2 | 5.4 | 4.4 | 2.1 |
| 8 | Ct | 36.4 | 27.9 | 28.3 | 29.0 | 32.2 | 33.0 | 34.9 | 31.9 | 32.3 | 35.0 |
| | SD | 0.3 | 0.4 | 0.5 | 0.3 | 0.3 | 0.3 | 0.5 | 0.4 | 0.4 | 0.4 |
| | ΔCt | 0.0 | 8.5 | 8.1 | 7.4 | 4.2 | 3.4 | 1.5 | 4.5 | 4.1 | 1.4 |
| 9 | Ct | 36.5 | 28.6 | 28.5 | 29.4 | 31.8 | 32.6 | 35.1 | 32.1 | 33.8 | 34.5 |
| | SD | 0.2 | 0.4 | 0.3 | 0.4 | 0.3 | 0.5 | 0.5 | 0.4 | 0.3 | 0.5 |
| | ΔCt | 0.0 | 7.9 | 8 | 7.1 | 4.7 | 3.9 | 1.4 | 4.4 | 2.7 | 2 |
| 10 | Ct | 36.7 | 27.6 | 28.1 | 28.8 | 31.7 | 32.8 | 35.4 | 31.4 | 33.1 | 34.9 |
| | SD | 0.4 | 0.3 | 0.4 | 0.3 | 0.5 | 0.3 | 0.4 | 0.3 | 0.5 | 0.4 |
| | ΔCt | 0.0 | 9.1 | 8.6 | 7.9 | 5 | 3.9 | 1.3 | 5.3 | 3.6 | 1.8 |

Based on the results of amplification of the b2M gene transcript site in cDNA samples obtained using one of the embodiments of the Medium according to the main aspect of the invention in the concentration ranges indicated in **Table 1,** in the Device for collecting, storing and transporting biological samples, at different storage periods at different temperature conditions, as well as on the basis of data from a comparative analysis of the average values of the expression of this gene in cDNA samples obtained using vacuum tubes BD Vacutainer^{®} Plus with EDTA on the day 0 of storage at room temperature, it was concluded that extracellular RNA is resistant to all tested climatic factors under specified conditions of use and the ability to preserve the sample prior to analysis. Separately, it should be noted that on the 15th day of storage of whole blood samples, the level of expression of the b2M gene in the Device proposed in the framework of this application according to the second aspect of the invention is higher or comparable to the expression of this gene on the 0th day of storage of blood in tubes with EDTA, which completely solves the problem of transporting extracellular RNA, the analysis of which is mainly carried out as part of a liquid biopsy in oncology, up to 15 days in a wide range of negative and positive temperatures to the place of high-tech research - to world-class genomic research centers.

The analysis of possible variants of media, as they are described in paragraph 1 of the claims, was also carried out for:
- confirmation of a higher concentration of extracellular DNA and RNA, which is detected during DNA / RNA isolation after centrifugation from blood samples in tubes according to the invention with an anticoagulant (for example, EDTA), cell membrane fixative and gel compared to tubes with **EDTA** without / with barrier gel and specialized tubes for stabilizing extracellular nucleic acids, in particular, with ***Cell-Free DNA BCT^{®}*** tubes manufactured by Streck (USA) with anticoagulant and cell membrane fixative without gel;
- confirmation of a higher concentration of extracellular DNA and RNA, which is found in DNA / RNA isolation after centrifugation and 1 freeze / thaw cycle of a blood sample in tubes according to the invention with an anticoagulant (for example, EDTA), cell membrane fixative and gel compared to tubes with **EDTA** without / with barrier gel and specialized tubes for stabilizing extracellular nucleic acids, in particular, with ***RNA Complete BCT^{®}*** tubes manufactured by Streck (USA) with anticoagulant and cell membrane fixative without gel without freezing cycles.

### Example 3. Stabilization of extracellular DNA and RNA using the Medium and Device for collecting, storing and transporting biological samples according to the present invention and commercially available analogues (comparative analysis)

### Blood sampling

Blood sampling from healthy donors was carried out on the basis of informed voluntary consent in the Device for collecting, storing and transporting biological samples according to the second aspect of the invention, which in this embodiment is a vacuum tube containing the Medium according to the main aspect of the invention, having the composition as indicated in **Table 1.** Within the scope of this study samples were intended for:
- checks for a higher concentration of extracellular DNA and RNA, which should be detected at shelf life 0 in the isolation of DNA / RNA after centrifugation from blood samples in tubes according to the invention with an anticoagulant (for example, EDTA), cell membrane fixative and gel compared to tubes with ***EDTA*** without / with berrier gel and specialized tubes for stabilization of extracellular nucleic acids, in particular, with ***Cell-Free DNA BCT^{®}*** tubes manufactured by Streck (USA) with anticoagulant and cell membrane fixative without gel;
- checking for a higher concentration of extracellular DNA and RNA, which should be detected at 0 shelf life during DNA / RNA isolation after centrifugation and 1 freeze / thaw cycle of a blood sample in tubes according to the invention with an anticoagulant (for example, EDTA), cell membrane fixative and gel according to the invention compared with ***EDTA*** tubes without / with barrier gel and specialized tubes for stabilization of extracellular nucleic acids, in particular, with ***RNA Complete BCT^{®}*** tubes manufactured by Streck (USA) with anticoagulant and cell membrane fixative without gel without freezing cycles.

To solve these problems, the following blood samples were taken, followed by centrifugation of the tubes within 1 hour after blood sampling:
∘ *2 test tubes with anticoagulant, cell membrane fixative and gel according to the present invention, size 16*×*100 from each donor, followed by storage at room temperature* +*(15-25)°C until centrifugation and further nucleic acid isolation* - samples *S30, S31;*
∘ *2 test tubes with anticoagulant, cell membrane fixative and gel according to the present invention, size 16*×*100 from each donor, followed by storage at room temperature* +*(15-25)°C until centrifugation and further nucleic acid isolation after 1 freeze*/*thaw cycle* - samples S32, S33;
∘ *2 tubes of 16*×*100 BD Vacutainer^{®} Plus EDTA tubes from each donor, kept on ice until centrifuged and then nucleic acid isolated* - samples S34, S35;
∘ *2 tubes of 16*×*100 BD* Vacutainer^{®} *PPT*^{™} *EDTA Separation Gel tubes from each donor, stored on ice until centrifugation and subsequent nucleic acid isolation -* samples S36, S37;
∘ *1 tube of Cell-Free DNA BCT*^{®} *manufactured by Streck (USA) with an anticoagulant and a cell membrane fixative without gel, 10 ml from each donor, followed by storage at room temperature* +*(15-25)°C until centrifugation and then isolation extracellular DNA* - sample S38;
∘ *1 tube* of RNA *Complete BCT*^{®} *manufactured by Streck (USA) with an anticoagulant and a cell membrane fixative without gel, 10 ml from each donor, followed by storage at room temperature* +*(15-25)°C until centrifugation and then isolation of extracellular RNA* - sample S39.

Blood samples were taken into vacuum tubes by venipuncture with standard double-ended needles. The vacuum tubes were filled to the mark, then the tube was removed from the needle holder / adapter and immediately mixed gently by gently inverting the tube 8-10 times (without shaking).

### Preparation of Plasma

### Samples S30-S33:

Within **1 hour** after taking venous blood, the tubes S30-S33 were centrifuged at room temperature +(15-25)°C for 15 minutes at 2800g, followed by placing the tubes for storage under different temperature conditions:
- at room temperature: *S30, S31;*
- in a freezer at a temperature of -20°C until the contents of the tube are completely frozen with further thawing at room temperature and subsequent smooth mixing 4-6 times or soft pipetting: *S32, S33.*

Next, the plasma was prepared for the study as follows:
- ½ volume of plasma was collected into a clean Eppendorf tube;
- centrifugation of the tested sample
   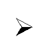 at +4°C for 10 minutes at 16000g in the case of samples S30 and S32, which were intended for extraction of extracellular DNA;
   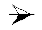 at room temperature for 15 minutes at 2800g in the case of samples S31 and S33, which were designed to isolate extracellular RNA;
- Carefully separate the supernatant, transfer it to a clean Eppendorf tube;
- extracellular DNA (tubes with samples S30, S32) and RNA (tubes with samples S31, S33) were isolated from plasma according to the respective manufacturer's instructions.

### Samples S34, S35:

Within **1 hour** after taking venous blood, centrifugation of tubes with samples S34, S35 was performed at room temperature +(15-25)°C for 20 minutes at low speed (900g), since blood centrifugation at high speed inevitably leads to the destruction of some cells and the nucleic acids they contain into plasma.

Next, plasma was collected and prepared, followed by isolation of extracellular DNA (tube with sample S34) similarly to tubes with samples S30, S32 and extracellular RNA (tube with sample S35) similarly to tubes with samples S31, S33 from plasma according to the relevant manufacturer's instructions.

### Samples S36, S37:

Within **1 hour** after taking venous blood, tubes with samples S36, S37 were centrifuged at room temperature +(15-25)°C for 20 minutes at 1500g, since this value of relative centrifugal acceleration is declared by the manufacturer as the maximum allowable (recommended range from 1100 to 1500g), in which there is no destruction of cells and the entry of nucleic acids contained in them into the plasma.

Next, plasma was collected and prepared, followed by isolation of extracellular DNA (tube with sample S36) similarly to tubes with samples S30, S32, S34 and extracellular RNA (tube with sample S37) similarly to tubes with samples S31, S33, S35 from plasma according to the relevant manufacturer's instructions.

### Samples S38, S39:

Within **1 hour** after taking venous blood, centrifugation was carried out for:
➢ tube with sample S38 intended for subsequent isolation of extracellular DNA, at room temperature +(15-25)°C for 10 minutes at 1600g;
➢ tube with sample S39 intended for subsequent extraction of extracellular RNA, at room temperature +(15-25)°C for 15 minutes at 1800g.

Next, plasma was collected and prepared, followed by isolation of extracellular DNA (tube with sample S38) similarly to tubes with samples S30, S32, S34, S36 and RNA (tube with sample S39) similarly to tubes with samples S31, S33, S35, S37 from plasma according to the relevant manufacturer's instructions.

### Extraction of Extracellular DNA

Isolation of extracellular DNA from plasma was performed using the MagMAX^{™} Cell-Free DNA Isolation Kit (ThermoFisher Scientific, USA) according to the methodology indicated in Example 1.

The presence of a fluorescence peak in the length range of fragments of 150-250 bps, characteristic of extracellular DNA, was assessed by capillary electrophoresis on TapeStation 2200 equipment (Agilent, USA) using ready-made reagent kits in accordance with the manufacturer's instructions and according to the methodology specified in the Example 1. Extracellular DNA concentration was measured using a Qubit 4.0 fluorimeter (ThermoFisher Scientific, USA) using the High Sensitivity dsDNA kit according to the protocol recommended by the manufacturer. For all samples, the concentration of extracellular DNA measured using a Qubit 4.0 fluorimeter corresponded to the concentration of extracellular DNA calculated from the area of the main fluorescence peak on the electropherogram obtained by capillary electrophoresis on TapeStation 2200 equipment, in the range of 150-250 bps fragment lengths characteristic of extracellular DNA. The results of determining the concentration of extracellular DNA, the isolation of which was carried out after centrifugation at 0 shelf life without freezing and with 1 freeze / thaw cycle from blood samples in tubes with anticoagulant, cell membrane fixative and gel according to the present invention in comparison with tubes with ***EDTA*** without / with separation gel and specialized tubes for stabilization of extracellular nucleic acids ***Cell-Free DNA BCT^{®}*** manufactured by Streck (USA) with anticoagulant and cell membrane fixative without gel without freezing cycles are shown in **Table 9.**

**Table 9. Results of determining the concentration of extracellular DNA released after centrifugation at 0 day of shelf life without freezing and with 1 freeze / thaw cycle from blood samples in tubes with anticoagulant, cell membrane fixative and gel according to the present invention in comparison with tubes with EDTA without / with barrier gel and specialized tubes for stabilizing extracellular nucleic acids Cell-Free DNA BCT^{®} with anticoagulant and cell membrane fixative without gel without freeze cycles.**

| Donor # | Characteristic | Tested Samples | | | | |
|---|---|---|---|---|---|---|
| | | Device with medium according to the main aspect of the invention | | Reference tubes | | |
| | | Tubes according to the invention *(S30)* | Tubes according to the invention after 1 cycle of freezing and thawing *(S32)* | BD Vacutainer^{®} Plus tubes with EDTA *(S34)* | $D Vacutainer^{®} PPT^{™} tubes with EDTA and barrier gel *(S36)* | Cell-Free DNA BCT^{®} tubes *(S38)* |
| 1 | The presence of a fluorescence peak in the length range of fragments of 150-250 bps, characteristic of extracellular DNA | + | + | + | + | + |
| | Extracellular DNA concentration, ng/ml plasma | 44 | 48 | 20 | 19 | 29 |
| | The ratio of the concentration of extracellular DNA in the device with the | 1.00 (S30/S30) | 0.92 (S30/S32) | 2.20 (S30/S34) | 231 (S30/S36) | 1.52 (S30/S38) |
| | medium according to the main aspect of the invention without (S30) and with 1 cycle of freezing and thawing (S32) to the concentration of extracellular DNA in the corresponding reference tube on day 0 of shelf life* | 1.09 (S32/S30) | 1.00 (S32/S32) | 2.40 (S32/S34) | 2.53 (S32/S36) | 1.65 (S32/S38) |
| 2 | The presence of a fluorescence peak in the length range of fragments of 150-250 bps, characteristic of extracellular DNA | + | + | + | + | + |
| | Extracellular DNA concentration, ng/ml plasma | 61 | 70 | 28 | 35 | 46 |
| | The ratio of the concentration of extracellular DNA in the device with the medium according to the main aspect of the invention without (S30) and with 1 | 1.00 (S30/S30) | 0.87 (S30/S32) | 2.18 (S30/S34) | 1.75 (S30/S36) | 1.32 (S30/S38) |
| | cycle of freezing and thawing (S32) to the concentration of extracellular DNA in the corresponding reference tube on day 0 of shelf life* | 1.15 (S32/S30) | 1.00 (S32/S32) | 2.50 (S32/S34) | 2.00 (s32/S36) | 1.52 (S32/S38) |
| 3 | The presence of a fluorescence peak in the length range of fragments of 150-250 bps, characteristic of extracellular DNA | + | + | + | + | + |
| | Extracellular DNA concentration, ng/ml plasma | 55 | 65 | 25 | 24 | 41 |
| | The ratio of the concentration of extracellular DNA in the device with the medium according to the main aspect of the invention without (S30) and with 1 | 1.00 (S30/S30) | 0.85 (S30/S32) | 2.20 (S30/S34) | 2.29 (S30/S36) | 1.34 (S30/S38) |
| | cycle of freezing and thawing (S32) to the concentration of extracellular DNA in the corresponding reference tube on day 0 of shelf life* | 1.18 (532/530) | 1.00 (S32/S32) | 2.60 (S32/S34) | 2.71 (S32/S36) | 1.58 (S32/S38) |
| 4 | The presence of a fluorescence peak in the length range of fragments of 150-250 bps, characteristic of extracellular DNA | + | + | + | + | + |

| Donor # | Characteristic | Tested Samples | | | | |
|---|---|---|---|---|---|---|
| | | Device with medium according to the main aspect of the invention | | Reference tubes | | |
| | Extracellular DNA concentration, ng/ml plasma | 85 | 102 | 35 | 39 | 51 |
| | The ratio of the concentration of extracellular DNA in the device with the medium according to the main aspect of the invention without (S30) and with 1 cycle of freezing and thawing (S32) to the concentration of extracellular DNA in the corresponding reference tube on day 0 of shelf life* | 1.00 (S30/S30) | 0.83 (S30/S32) | 2.43 (S30/S34) | 2.18 (S30/S36) | 1.67 (S30/S38) |
| | DNA in the corresponding reference tube at 0 shelflife* | 1.20 (S32/S30) | 1.00 (S32/S32) | 2.91 (S32/S34) | 2.61 (S32/S36) | 2.00 (S32/S38) |
| 5 | The presence of a fluorescence peak in the length range of fragments of 150-250 bps, characteristic of extracellular DNA | + | + | + | + | + |
| | Extracellular DNA concentration, ng/ml plasma | 38 | 48 | 17 | 16 | 32 |
| | The ratio of the concentration of extracellular DNA in the device with the medium according to the main aspect of the invention without (S30) and with 1 cycle of freezing and thawing (S32) to the concentration of extracellular DNA in the corresponding reference tube on day 0 of shelf life* | 1.00 (S30/S30) | 0.79 (S30/S32) | 223 (S30/S34) | 2.37 (S30/S36) | 1.19 (S30/S38) |
| | | 1.26 (S32/S30) | 1.00 (S32/S32) | 2.82 (S32/S34) | 3.00 (S32/S36) | 1.50 (S32/S38) |

| | | | | | | |
|---|---|---|---|---|---|---|
| * day of venous blood sampling in vacuum tubes by venipuncture | | | | | | |

As a result of the testing of one of the embodiments of the Medium according to the main aspect of the invention, containing a stabilizing solution and a thixotropic barrier gel in the Device for collecting, storing and transporting biological samples in the concentration ranges indicated in **Table 1,** a higher concentration of extracellular DNA was confirmed, which is detected in DNA extraction after centrifugation without freezing and with 1 freeze/thaw cycle of a blood sample in test tubes according to the invention compared to:
- *EDTA* tubes without gel - increase of up to 2.43 and 2.91 times, respectively;
- *EDTA* tubes with barrier gel - increase of up to 2.37 and 3,00 times, respectively;
- ***Cell-Free DNA BCT*^{®}** tubes of Streck (CIIIA) with anticoagulant and gel free fixating agent of cell membrane - increase of up to 1.67 and 2.00 times, respectively.

### RNA extraction, cDNA preparation and real-time PCR

Extraction of RNA from plasma for further formulation of the reverse transcription reaction was carried out using the Proba-NK kit (DNA-Technology LLC, Russia) in three repetitions according to the methodology indicated in Example 2.

A qualitative assessment of the purity of circulating extracellular RNA preparations was performed by measuring the optical density of aliquots of the obtained samples at wavelengths of 260 and 280 nm on a NanoDrop 2000 spectrophotometer in accordance with the manufacturer's instructions for use. The absorbance ratio 260/280 for all analyzed samples was 1.8-2.0.

The reverse transcription reaction was carried out using a reverse transcription reagent kit (OOO DNA-Technology, Russia) according to the methodology indicated in Example 2. The cDNA samples obtained as a result of reverse transcription were used to assess the expression level of the housekeeping gene - beta-2-microglobulin (b2M) real-time PCR.

The analysis of the obtained results was carried out using the software of the cycler used. Additionally determined:
- value ΔCt¹ = Ct^{reference sample} (average threshold cycle of a cDNA sample prepared using the BD Vacutainer^{®} Plus EDTA Comparison Tube, BD Vacutainer^{®} PPT^{™} EDTA with Separation Gel, or RNA Complete *BCT*^{®} with Anticoagulant and Cell Membrane Fixator without gel at day 0 storage) - sample Ct (average threshold cycle of the studied cDNA sample obtained using one of the embodiments of the Medium according to the main aspect of the invention in the Device for collecting, storing and transporting biological samples after centrifugation at room temperature +(15-25)°C at 0 storage period without freezing);
- value Δ**Ct²** = **Ct**^{reference sample} (average threshold cycle of a cDNA sample prepared using the BD Vacutainer^{®} Plus EDTA Comparison Tube, BD Vacutainer^{®} PPT^{™} EDTA with Separation Gel, or RNA Complete *BCT*^{®} with Anticoagulant and Cell Membrane Fixator without gel on day 0 of storage) - sample Ct (average threshold cycle of the studied cDNA sample obtained using one of the embodiments of the Medium according to the main aspect of the invention in the Device for collecting, storing and transporting biological samples after centrifugation at room temperature +(15-25)°C for day 0 of shelf life with 1 freeze cycle / defrosting);
- the ratio of extracellular RNA concentration in the device according to the main aspect of the invention in relation to the extracellular RNA concentration in the corresponding reference tube at 0 storage period without freezing or with 1 freeze/thaw cycle = 1,933^{ΔCt1} 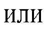 1,933^{Δ**Ct2**} accordingly, where 1,933 - efficiency coefficient of PCR by titration method.

Examples of the results of determining the concentration of extracellular RNA, the isolation of which was carried out after centrifugation at room temperature +(15-25)°C at 0 shelf life without freezing and with 1 freeze / thaw cycle from blood samples in tubes with anticoagulant, cell membrane fixative and gel according to the present invention in comparison with tubes with EDTA without / with separation gel and specialized tubes for stabilization of extracellular nucleic acids RNA Complete BCT^{®} manufactured by Streck (USA) with anticoagulant and cell membrane fixative without gel without freezing cycles are shown in **Table 10**.

**Table 10. Results of determining the concentration of extracellular RNA, the extraction of which was carried out after centrifugation at room temperature +(15-25)°C on day 0 of storage period without freezing and with 1 freeze / thaw cycle from blood samples in test tubes with anticoagulant, cell membrane fixative and gel according to the present invention compared to EDTA tubes without/with separation gel and specialized tubes for stabilizing extracellular nucleic acids RNA Complete BCT^{®} with anticoagulant and cell membrane fixative without gel without freezing cycles.**

| Donor # | Charactexitic | Tested Samples | | | | |
|---|---|---|---|---|---|---|
| | | Device with medium according to the main aspect of the invention | | Reference Tubes | | |
| | | Tubes according to the invention *(S31)* | Tubes according to the invention after 1 cycle of freezing and thawing *(S33)* | BD Vacutainer^{®} Plus tubes with EDTA *(S35)* | BD Vacutainer^{®} PPT^{™} tubes with EDTA and barrier gel *(S37)* | RNA Complete BCT^{®} tubes *(S39)* |
| 1 | Ct | 27.2 | 25.4 | 36.8 | 37.0 | 32.4 |
| | SD | 0.4 | 0.5 | 0.6 | 0.7 | 0.6 |
| | ΔCt¹ | - | - | 9.6 | 9.8 | 5.2 |
| | 1,933^ΔCt¹ | - | - | 560 | 638 | 31 |
| | ΔCt² | - | - | 11.4 | 11.6 | 7 |
| | 1,933^ΔCt² | - | - | 1833 | 2091 | 102 |
| 2 | Ct | 28.1 | 27.8 | 36.7 | 37.2 | 33.4 |
| | SD | 0.5 | 0.5 | 0.7 | 0.7 | 0.6 |
| | ΔCt¹ | - | - | 8.6 | 9.1 | 5.3 |
| | 1,933^ΔCt¹ | - | - | 290 | 402 | 33 |
| | ΔCt² | - | - | 8.9 | 9.4 | 5.6 |
| | 1,933^ΔCt² | - | - | 353 | 490 | 40 |
| 3 | Ct | 27.8 | 263 | 35.9 | 36.5 | 33.1 |
| | SD | 0.5 | 03 | 0.7 | 0.6 | 0.6 |
| | ΔCt¹ | - | - | 8.1 | 8.7 | 5.3 |
| | 1,933^ΔCt¹ | - | - | 208 | 309 | 33 |
| | ΔCt² | - | - | 9.6 | 10.2 | 6.8 |
| | 1,933^ΔCt² | - | - | 559 | 831 | 88 |
| 4 | Ct | 28.0 | 26.9 | 37.5 | 37.4 | 33.6 |
| | SD | 0.4 | 0.4 | 0.6 | 0.7 | 0.4 |
| | ΔCt¹ | - | - | 9.5 | 9.4 | 5.6 |
| | 1,933^ΔCt¹ | - | - | 533 | 490 | 40 |
| | ΔCt² | - | - | 10.6 | 10.5 | 6.7 |
| | 1,933^ΔCt² | - | - | 1082 | 1013 | 83 |
| 5 | Ct | 26.3 | 25.2 | 37.2 | 37.4 | 32.0 |
| | SD | 03 | 0.4 | 0.6 | 0.7 | 0.4 |
| | ΔCt¹ | - | - | 10.9 | 11.1 | 5.7 |
| | 1,933^ΔCt¹ | - | - | 1318 | 1504 | 43 |
| | ΔCt² | - | - | 12 | 12.2 | 6.8 |
| | 1,933^ΔCt² | - | - | 2721 | 3105 | 88 |

As a result of the testing of one of the embodiments of the Medium according to the main aspect of the invention, containing a stabilizing solution and a thixotropic barrier gel in the Device for collecting, storing and transporting biological samples in the concentration ranges indicated in **Table 1,** geometric mean values were calculated for the values of 1.933^ΔCt1 and 1.933^ΔCt2 obtained from the analysis of 5 blood samples from healthy donors **(Table 10),** thus confirming the higher concentrations of extracellular RNA found in RNA isolation after centrifugation without freezing and with 1 freeze/thaw cycle of the blood sample in test tubes according to the invention compared to:
- *EDTA* tubes without gel - an average increase of 464 and 1013 times, respectively;
- *EDTA* tubes with barrier gel - an average increase of 567 and 1317 times, respectively;
- *RNA Complete BCT*^{®} tubes of Streck (USA) with anticoagulant and cell membrane fixative without gel - an average increase of 36 and 76 times, respectively.

### BIBLIOGRAPHY

1 La Verde M., De Falco L., Torella A., Savarese G, Savarese P, Ruggiero R, Conte A, Fico V, Torella M, Fico A. Performance of cell-free DNA sequencing-based non-invasive prenatal testing: experience on 36,456 singleton and multiple pregnancies // BMC Med. Genomics.- 2021.- Vol. 14, 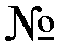 1.- P. 93.
2 Oellerich M., Schiitz E., Beck J., Walson P.D. Circulating cell-free DNA-diagnostic and prognostic applications in personalized cancer therapy // Ther. Drug Monit.- 2019.-Vol. 41, Nº 2.- P. 115-120.
3 Jaikaransingh V., Kadambi P.V. Donor-derived cell-free DNA (ddcf-DNA) and acute antibody-mediated rejection in kidney transplantation // Medicina (Kaunas).- 2021.-Vol. 57, Nº 5.- P. 436.
4 Haller N., Helmig S., Taenny P., Petry J., Schmidt S., Simon P. Circulating, cell-free DNA as a marker for exercise load in intermittent sports // PLoS One.- 2018.- Vol. 13, Nº 1.- e0191915.
5 Hammad R., Eldosoky M.A.E.R., Fouad S.H., Elgendy A., Tawfeik A.M., Alboraie M., Abdelmaksoud M.F. Circulating cell-free DNA, peripheral lymphocyte subsets alterations and neutrophil lymphocyte ratio in assessment of COVID-19 severity // Innate Immun.-2021.- Vol. 27, Nº 3.- P. 240-250.
6 Lis J.T., Schleif R. Size fractionation of double-stranded DNA by precipitation with polyethylene glycol // Nucleic Acids Res.- 1975 - Vol. 2, Nº 3.- P. 383-389.
7 Gerald Litwack. Human Biochemistry / 1st Edition, 2018 / Chapter 14. Metabolism of Fat, Carbohydrate, and Nucleic Acids: Nucleic Acid Metabolism.- P. 415-420 // Imprint: Academic Press.- 778 p.
8 Gorokhova S.G., Atkov O.Yu., Gorbachev A.Yu., Generozov E.V., Alchinova I.B., Polyakova M.V., Karganov M.Yu. Change of transcription level of photoreceptor-specific CRX gene in the peripheral blood of the participants of an arctic world oceanic international flight // The Russian Annals of Ophthalmology = Vestnik Oftalmologii.-2021.- V. 137, Nº 2.- P. 5-11 (In Russ.). https://doi.org/10.17116/oftalma20211370215

## Claims

1. Medium for collection, storage, and transportation of biological samples, comprising a barrier gel and a stabilizer which in turn comprises at least one cell membrane fixing agent and at least one anticoagulant, **characterized in that**:
a. The biological sample comprising the extracellular nucleic acids to be preserved is blood or plasma; and the extracellular nucleic acids are extracellular DNA or RNA;
b. The barrier gel comprises thixotropic gel with a density in the range from 1.025 to 1.065 g/cm³, is chemically inert with respect to biological samples, and has sufficient viscosity so that under centrifugal forces in the range of 2000 to 3600 g it will flow and form the desired barrier between plasma and blood cells;
c. The cell membrane fixing agent is selected from 2-bromo-2-nitropropane-1,3-diol, dimethylol urea, sodium hydroxymethylglycinate, diazolidinyl urea, dimethyl-dimethylol-hydantoin, imidazolidinyl urea, 1,3,5-tris(hydroxyethyl)-s-triazine, 1,3-bis(hydroxymethyl)-5,5-dimethylimidazolidine-2,4-dione, dimethylol-5,5-dimethylhydantoin, or combinations thereof; releases free aldehyde and has the properties of a preservative; and
d. The anticoagulant is selected from ethylene glycol tetraacetic acid (EGTA) and its salts, sodium citrate, ethylenediaminetetraacetic acid (EDTA) and its salts, sodium fluoride or combinations thereof; and acts as an ionic stabilizing and/or chelating agent.

2. Medium, according to claim 1, where the fixing agent can mostly be from 0.2% to 63.4% of the total formulation weight.

3. Medium, according to any of the previous claims, where the anticoagulant can mostly be from 0.1% to 28.3% of the total formulation mass.

4. Medium, according to any of the previous claims, where the stabilizer may not necessarily include a quenching agent in an amount sufficient to react with any free aldehyde that may be present before or after sampling and which is formed from the fixing agent to form a reaction product that will not have a denaturing effect on proteins in biological samples, while the content of the quenching agent can mainly be from 0% to 7.8% by weight of the entire formulation, and wherein: i) a compound including at least one functional group capable of reacting with an electron-deficient functional group of an aldehyde can be used as a quenching agent and ii) ethylenediamine, amino acetic acid, lysine, arginine, adenine, guanine, cytosine, thymine, tryptophan, tyrosine, phenylalanine, omithine, S-adenosylmethionine, alanine, cysteine, glutamic acid, aspartic acid, histidine, leucine, lysine, proline, serine, threonine, homocysteine, nicotinamide, or combinations thereof can be used as a quenching agent.

5. Medium, according to any of the previous claims, where the stabilizer may not necessarily include additional non-ionic stabilizing agents at a concentration of 0% to 18.5% by weight of the entire formulation, which dissolve in an aqueous solution without the formation of ions and can be selected from the group of polyols (polyhydric alcohols), such as sucrose, lactose, trehalose, melibiose, mannitol, or combinations thereof.

6. Medium, according to any of the previous claims, where the stabilizer may not necessarily include enzyme inhibitors - nucleases and / or proteases, including inhibitors of metabolic enzymes and inhibitors of phosphatase, which prevent the degradation of nucleic acids and ensure their stabilization, as well as inhibitors of apoptosis, including caspase inhibitors, including in combination with N,N-dialkylpropanamide, for example, with N,N-dimethylpropanamide (DMPA), or butanamide, which prevent cell apoptosis through a caspase-dependent pathway, and transcription inhibitors, which are designed to maintain the basic level of RNA expression at the time of blood sampling by inhibiting the synthesis of new RNA transcripts, including mRNA, microRNA, IncRNA, piRNA, YRNA, circRNA and other ncRNA: i) enzyme inhibitors can be selected from the groups of compounds, including but not limited to diethyl pyrocarbonate, ethanol, auryl tricarboxylic acid (ATA), glyceraldehydes, formamide, bentonite, ammonium sulfate, dithiothreitol (DTT), beta-mercaptoethanol, cysteine, dithioerythritol, tris(2-carboxyethyl)phosphine hydrochloride or combinations thereof; ii) inhibitors of metabolic enzymes can be selected from the groups of compounds, including but not limited to glyceraldehyde, dihydroxyacetone phosphate, glyceraldehyde-3-phosphate, 1,3-bisphosphoglycerate, 3-phosphoglycerate, 2 phosphoglycerate, phosphoenolpyruvate, pyruvate and glycerate dihydroxyacetate, potassium oxalate or combinations thereof; iii) protease inhibitors can be selected from the groups of compounds, including but not limited to antipain, chymostatin, elastatinal, phenylmethylsulfonyl fluoride (PMSF), APMSF, TLCK, TPCK, leupeptin, soybean trypsin inhibitor, indoleacetic acid (IAA), E64, pepstatin, 1,10-phenanthroline, phosphoramodone, amastatin, bestatin, diprotin A, diprotin B, alpha-2-macroglobulin, pancreatic protease inhibitor, ovostatin, cystatin, protease inhibitor, or combinations thereof; iv) phosphatase inhibitors can be selected from the groups of compounds, including but not limited to caliculin A, nodularin, NIPP-1, microcystin LR, tautomycin, okadaic (okadaic) acid, cantharidin, fostriyetsin, endothall, cyclosporin A, FK 506/immunophilin complexes, cypermethrin, deltamethrin, fenvalerate, defostatin, mpV (pic) DMHV, sodium orthovanadate, or combinations thereof; v) the content of enzyme inhibitors - nucleases and / or proteases, including inhibitors of metabolic enzymes and phosphatase inhibitors, can mainly range from 0% to 25.8% by weight of the entire formulation.

7. Medium, according to any of the previous claims, where the stabilizer may optionally contain, at a concentration of 0% to 5.4%, one or more cell permeability enhancing agents, which may be selected from the following compounds or combinations thereof: DMSO (dimethyl sulfoxide), ethylene glycol, glycerin, cellosolves, ethylene glycol dimethyl ether, phenoxyethanol, Triton X 100, Triton X 705 (non-ionic detergent), 1-methyl-2-pyrrolidinone, Tween 20, Tween 40 (non-ionic detergent), Brij 35 (non-ionic detergent), polyoxyethylene ester (Polyox), monensin, monactin, pentachlorophenol, 2,4-dinitrophenol, saponin, SDS (sodium dodecyl sulfate).

8. Medium, according to any of the previous claims, where the stabilizer may optionally contain the following compounds or combinations thereof at a concentration of 0% to 6.2%: i) proteins such as: biotin, albumins: egg, bovine, including bovine serum albumin - BSA, gelatin and similar compounds; ii) additional nucleic acid stabilizers such as: guanidine hydrochloride, polycations such as polyethyleneimine and similar compounds; iii) antioxidants/reductants such as Trolox, α-tocopherol, nicotinamide and similar compounds; iv) nucleic acid dyes such as: DAPI (diamidino-2-phenylindole), propidium iodide, fluorescein diacetate and similar compounds; v) antibiotics and other additional components, including but not limited to doxycycline, sulfasalazine, curcumin, 6-aminocaproic acid, minocycline, chitosan, phytic acid, β-sitosterol, c-AMP, polylysine, biochanin A, demeclocycline, chlortetracycline, oxytetracycline, cyclohexamide, rifampicin, soy milk, suramin, N-butyric acid, penicillamine, N-acetylcysteine, benzamidine, 2 aminoethylbenzylsulfonyl fluoride (AEBSF).

9. Medium, according to any of the previous claims, where the pH of the stabilizer can be, depending on the combination of its constituent agents and additional components, from 4 to 10, mostly from 6 to 8.

10. Device for collecting, storing and transporting biological specimen containing the medium according to claim 1 to 9, which comprises a test tube with the medium according to claims 1 to 9, provided that the stabilizer containing the cell membrane fixing agent, which is part of the medium according to claims 1 to 9, is located above the thixotropic gel and / or the specified thixotropic gel is located under the specified stabilizer.

11. Device, according to claim 10, comprising vacuum tubes, including sterile ones, which may contain a vacuum intended for aspiration of a biological sample, while vacuum tubes may mostly have, without the limitation, a nominal capacity of 9.0 ml, 8.5 ml, 8.0 ml, 7.5 ml, 7.0 ml (size 16×100 mm), 6.0 ml, 5.5 ml, 5.0 ml (size 13×100 mm), 2.0 ml, 2.5 ml, 3.0 ml, 3.5 ml, 4.0 ml, 4.5 ml (13×75 mm).

12. Device, according to claims 10 or 11, in which the content of the separation gel is mostly from 0.3 g to 2.5 g per tube, without the limitation.

13. Use of device of claims 10 to 12, which comprises the medium of claims 1 to 9, for stabilizing extracellular nucleic acids for up to 15 days at temperatures ranging from -20°C to +37°C and up to 12 months at temperatures ranging from -86°C to -20°C.

14. Use, according to claim 13, **characterized in that** is carried out through the interaction of a biological sample located in the device according to claims 10 to 12, with a cell membrane fixing agent that is part of the stabilizing medium solution according to claims 1 to 9, and a separating gel, which after centrifugation for 10-30 minutes at 2000-3600g creates a separating barrier between the supernatant and cells, mainly plasma and blood cells, making the nucleic acids in the plasma sample suitable for extraction and subsequent analysis, including but not limited to PCR and sequencing.

15. Use, according to claims 13 or 14, **characterized by** taking a biological sample with the possibility of storage and transportation without centrifugation up to 24 hours after taking at a temperature ranging from +4°C to +37°C, followed by centrifugation and further storage and transportation from 5 to 15 days at temperatures from -20°C to +37°C with the possibility of repeated freezing / thawing of the sample after centrifugation with storage at temperatures from -86°C to -20°C for up to 12 months.

16. Use, according to claims 13 to 15, **characterized by** the possibility of storing and transporting a biological sample without centrifugation from 5 to 15 days after taking at a temperature ranging from +4°C to +37°C and centrifugation immediately before the collection of the supernatant, mainly plasma, to remove cells, extracellular structures and cellular debris to obtain cell-free plasma.

17. Use, according to claims 13 to 16, **characterized by** the possibility of storing and transporting a biological sample without centrifugation from 5 to 15 days after taking at a temperature ranging from +4°C to +37°C while preserving extracellular nucleic acids, as well as circulating cells and / or extracellular structures, if necessary.
